# EUROPEAN PATENT APPLICATION

(11) **EP 3 970 726 A1**
(43) Date of publication of application: **23.03.2022**
(21) Application number: 21191684.6
(22) Date of filing: 24.06.2011
(51) Int. Cl.: A61K 31/575, A61K 31/573, A61K 31/58, A61K 9/14, A61K 9/16, A61P 29/00, A61K 9/00, A61K 47/36, A61P 1/00

(54) **METHODS OF TREATMENT FOR ESOPHAGEAL INFLAMMATION**

(30) Priority: 24.06.2010 US 35840110 P; 28.06.2010 US 35931110 P; 24.11.2010 US 41708510 P; 06.05.2011 US 201161483580 P
(62) Divisional of application: 11799004.4
(71) Applicant: ViroPharma Biologics LLC, Lexington, MA 02421 (US)
(72) Inventor: PHILLIPS, Elaine, San Diego,, 92130 (US); HILL, Malcolm, San Diego,, 92130 (US)
(74) Representative: Carpmaels & Ransford LLP

(57) **Abstract**

Provided herein are methods for treating gastrointestinal inflammation, for example, esophageal inflammation, or reduction of eosinophilic infiltration of the esophagus and/or reducing local and systemic exposure and/or side effects resulting therefrom. Provided herein are methods for diagnosing gastrointestinal inflammation, for example, esophageal inflammation, or reduction of eosinophilic infiltration of the esophagus. Also provided herein are methods for inducing remission of eosinophilic infiltration of the esophagus

## Description

### CROSS REFERENCE

This application claims the benefit of U.S. Provisional Application No. 61/358,401, filed June 24, 2010; U.S. Provisional Application No. 61/359,311, filed June 28, 2010; U.S. Provisional Application No. 61/417,085, filed November 24, 2010; U.S. Provisional Application No. 3261/483,580, filed May 6, 2011; which are entirely incorporated herein by reference.

### BACKGROUND OF THE INVENTION

Esophageal inflammation disorders are gaining increased recognition in both adults and children. One example is eosinophilic esophagitis (EE or EoE), which is an emerging, and fast-growing disorder and can be characterized by high levels of eosinophils in the esophagus, and/or basal zone hyperplasia. Diagnosis of EE (or EoE) is often made based on the finding of 15 to 20 or more to 24 or more eosinophils per high power field (eos/hpf) within esophageal mucosal biopsies.

In parallel with other atopic disorders, the incidence of EE (or EoE) appears to be increasing. Symptoms of EE (or EoE) include, for example, abdominal pain, chest pain, choking, difficulty swallowing, failure to thrive, nausea, reflux not relieved by standard anti-flux therapy, skin rash or hives, vomiting, and weight loss. In one series, 15% of EE (or EoE) patients had concurrent developmental delay.

Although EE (or EoE) is becoming more frequently diagnosed throughout developing countries many aspects of the disease remain unclear including its etiology, natural history and optimal therapy. For example, the overlap of gastroesophageal reflux disease ("GERD") and EE (or EoE) symptoms is common. The common occurrence regarding misdiagnosis of EE (or EoE) for GERD often results in delayed treatment for patients with EE (or EoE).

### SUMMARY OF THE INVENTION

Provided herein are orally administered compositions comprising an effective amount of a corticosteroid for use in reducing eosinophilic infiltration of the esophagus in an individual in need thereof, or for inducing remission of eosinophilic infiltration of the esophagus in an individual in need thereof.

In one embodiment, the invention encompasses methods for reducing eosinophilic infiltration of the esophagus in an individual in need thereof, comprising orally administering to the individual a therapeutically effective amount of a corticosteroid. In one embodiment, the invention encompasses methods for inducing remission of eosinophilic infiltration of the esophagus in an individual in need thereof, comprising orally administering to the individual a therapeutically effective amount of a corticosteroid.

In another embodiment, the invention encompasses methods for treating esophageal inflammation in an individual in need thereof, comprising: (a) orally administering to the individual a first daily amount of corticosteroid in an amount effective to reduce eosinophilic infiltration of the esophagus; and subsequently (b) orally administering to the individual a second daily amount of corticosteroid in an amount effective to maintain a reduced level of eosinophilic infiltration of the esophagus.

In another embodiment, the invention encompasses methods for treating esophageal inflammation in an individual in need thereof, comprising: (a) orally administering to the individual a first daily amount of corticosteroid in an amount effective to reduce eosinophilic infiltration of the esophagus; (b) orally administering to the individual a second daily amount of corticosteroid in an amount effective to further reduce eosinophilic infiltration of the esophagus; and subsequently (c) orally administering to the individual a third daily amount of corticosteroid in an amount effective to maintain a reduced level of eosinophilic infiltration of the esophagus.

All embodiments described herein as methods of treatment are understood to include descriptions of compositions for use in such therapies as well.

### INCORPORATION BY REFERENCE

All publications and patent applications mentioned in this specification are herein incorporated by reference in their entirety to the same extent as if each individual publication or patent application was specifically and individually indicated to be incorporated by reference.

### BRIEF DESCRIPTION OF THE DRAWINGS

Figure 1 illustrates one embodiment of the invention comprising histologic response to oral viscous budesonide ("OVB", which is used interchangeably herein with oral budesonide suspension ("OBS")) treatment according to Example 1.
Figure 2 illustrates one embodiment of the invention comprising histologic remission after OVB treatment according to Example 1
Figure 3 illustrates one embodiment of the invention comprising peak pre- and post-treatment eosinophil counts by esophageal site according to Example 1. Figure 3a relates to placebo, Figure 3b relates to low dose OVB, Figure 3c relates to medium dose OVB, and Figure 3d relates to high dose OVB.
Figure 4 illustrates one embodiment of the invention comprising morning cortisol levels for each treatment group according to Example 1.
Figure 5 illustrates one embodiment of the invention comprising response to OVB treatment according to Example 1.
Figure 6 illustrates one embodiment of the invention comprising LP Score response to OVB treatment according to Example 1.
Figure 7 illustrates subjects achieving response (response for histology variable was defined as post-treatment peak eosinophil count ≤ 6/HPF; response for all other variables defined as a ≥ 50% reduction from baseline score) and remission (remission for histology variable was defined as post-treatment peak eosinophil count ≤ 1/HPF; remission for all other variables defined as a post-treatment score of 0) for selected study endpoints.
Figure 8 illustrates one embodiment of the invention comprising mean plasma concentration over time after OVB treatment according to Example 1.
Figure 9 illustrates one embodiment of the invention comprising mean plasma concentration over time according to age strata after OVB treatment according to Example 1.
Figure 10: illustrates pre and post-treatment esophageal biopsy results of one embodiment of a therapy described herein s (Distal 200x).
Figure 11 illustrates age-stratified mean plasma budesonide concentration over time.

### DETAILED DESCRIPTION OF THE INVENTION

The invention encompasses methods for reducing, including inducing remission of, eosinophilic infiltration of the esophagus in an individual in need thereof, comprising orally administering to the individual an effective amount of a corticosteroid.

In some embodiments, the individual is suffering from (and/or the therapies provided herein are for treating) eosinophilic esophagitis (EE or EoE), inflammatory bowel diseases involving the esophagus, Crohn's disease, celiac disease, proximal gastrointestinal pathology (*e*.*g*., in individuals suffering from hypofunctioning gallbladder), eosinophilic gastrointestinal inflammation, celiac disease, eosinophilic duodenitis, duodenal eosinophilia, functional dyspepsia, intermediate esophagitis, epithelial hyperplasia, basal cell hyperplasia, elongated papillae, dilated vessels in papillae, fungal esophagitis (*e*.*g*., Candida, turolopsis, histoplasma *Aspergillus*, etc.), viral esophagitis (*e*.*g*., HSV, CMV, V2V), bacterial esophagitis (*e*.*g*., tuberculosis, actinomycosis, syphilis), corrosive esophagitis, radiation esophagitis, chemotherapy esophagitis, eosinophilic gastric outlet obstruction and related inflammation, graft vs. host disease, a skin disease with esophageal involvement (*e*.*g*., bullous pemphigoid, pemphigus vulgaris, epidermolysis bollosa, Stevens-Johnson syndrome), Behçet's disease, sarcoidosis, idiopathic esophagitis, eosinophilic gastritis, Menetrier's disease, parasitic gastritis, lymphocytic esophagitis, inflammatory bowel disease-associated esophagitis, parasitic gastritis, esophageal inflammation secondary to caustic/irritant ingestion, persistent/recurrent esophageal strictures of any cause and including caustic/irritant ingestion, pill-induced esophagitis, a systemic disease, a congenital disease, Epidermolysis bullosa, post-surgery inflammation, dysphagia, heartburn/regurgitation, nausea/vomiting, pain, anorexia/early satiety, gastrointestinal bleeding, lamina propria fibrosis, food sensitization, extracellular eosinophil granules in the esophagus, eosinophilic microabscesses in the esophagus, surface layering of eosinophils in the esophagus, or gastro enteritis.

In other embodiments, the individual is suffering from a gastrointestinal disorder such as a stomach or duodenal ulcer, a hyperactive acidic discharge disorder, such as Zollinger-Ellison syndrome, or a laryngeal disorder, Barrett's Esophagus, gastroesophageal reflux disease (GERD), nonerosive reflux disease (NERD), or erosive esophagitis.

In other embodiments, the individual is suffering from eosinophilic esophagitis (EE or EoE).

In one embodiment, when eosinophilic infiltration of the esophagus is reduced, the individual has 1 or fewer eosinophils per high power field in one or more sites of the esophageal mucosa. In some embodiments, such reducing eosinophilic infiltration of the esophagus includes inducing remission of eosinophilic infiltration of the esophagus. Sites of the esophageal mucosa include the distal esophageal mucosa, the mid esophageal mucosa, and/or the proximal esophageal mucosa.

In one embodiment, when eosinophilic infiltration of the esophagus is reduced, the individual has 1 or fewer eosinophils per high power field in each of the proximal, mid and distal esophageal mucosa. In some embodiments, such reducing eosinophilic infiltration of the esophagus includes inducing remission of eosinophilic infiltration of the esophagus.

In another embodiment, when eosinophilic infiltration of the esophagus is reduced, the individual has 1 or fewer eosinophils per high power field in two of the proximal, mid and distal esophageal mucosa, i.e., in the proximal and mid esophageal mucosa, in the proximal and distal mucosa, or in the mid and distal esophageal mucosa. In some embodiments, such reducing eosinophilic infiltration of the esophagus includes inducing remission of eosinophilic infiltration of the esophagus.

In one embodiment, when eosinophilic infiltration of the esophagus is reduced, the individual has 6 or fewer eosinophils per high power field in one or more of the proximal, mid, and/or distal esophagus (e.g., esophageal mucosa). In some embodiments, when eosinophilic infiltration of the esophagus is reduced, the individual has 6 or fewer eosinophils per high power field in each of the proximal, mid, and distal esophagus (e.g., esophageal mucosa). In one embodiment, when eosinophilic infiltration of the esophagus is reduced, the individual has 1 or fewer eosinophils per high power field in the distal esophageal mucosa and 6 or fewer eosinophils per high power field in the mid esophageal mucosa and the proximal esophageal mucosa. In another embodiment, when eosinophilic infiltration of the esophagus is reduced, the individual has 1 or fewer eosinophils per high power field in the distal esophageal mucosa and the mid esophageal mucosa, and 6 or fewer eosinophils per high power field in the proximal esophageal mucosa. In one embodiment, when eosinophilic infiltration of the esophagus is reduced, the individual has 6 or fewer eosinophils per high power field in the distal esophageal mucosa and 1 or fewer eosinophils per high power field in the mid esophageal mucosa and the proximal esophageal mucosa. In another embodiment, when eosinophilic infiltration of the esophagus is reduced, the individual has 6 or fewer eosinophils per high power field in the distal esophageal mucosa and the mid esophageal mucosa, and 1 or fewer eosinophils per high power field in the proximal esophageal mucosa. In another embodiment, when eosinophilic infiltration of the esophagus is reduced, the individual has 6 or fewer eosinophils per high power field in the distal esophageal mucosa, the mid esophageal mucosa, and the proximal esophageal mucosa. In some embodiments, such reducing eosinophilic infiltration of the esophagus includes inducing remission of eosinophilic infiltration of the esophagus. In another embodiment, when eosinophilic infiltration of the esophagus is reduced, the individual has 1 or fewer eosinophils per high power field in the distal esophageal mucosa, the mid esophageal mucosa, and the proximal esophageal mucosa. In some embodiments, such reducing eosinophilic infiltration of the esophagus includes inducing remission of eosinophilic infiltration of the esophagus. In one embodiment, a response (i.e., reduction) in eosinophilic infiltration of the esophagus (or symptoms thereof) comprises a reduction of eosinophils per high power field in the esophagus (e.g., a statistically significant reduction, such as a statistically significant reduction to 6 or fewer eosinophils per high power field) (e.g., in one or more of the proximal, mid, and/or distal esophageal mucosa); and/or a reduction in symptom score (e.g., a statistically significant reduction as determined according to any suitable process, such as any symptom scoring process described herein; such as by an at least 50% symptom score reduction).

Reduction of eosinophilic infiltration of the esophagus (including, in some embodiments, e.g., inducing remission) can optionally be determined and can be performed by methods known or developed by those of skill in the art, and, for example, can be based on a clinical symptom score, an epithelial score, and/or a lamina propria ("LP") score. In some embodiments, reduction of eosinophilic infiltration of the esophagus is determined based on eosinophil count as described in multiple embodiments above and herein. In other embodiments, reduction of eosinophilic infiltration of the esophagus is determined based on a clinical symptom score, an epithelial score, an endoscopy score, and/or a lamina propria ("LP") score. In other embodiments, reduction of eosinophilic infiltration of the esophagus is determined based on eosinophil count as described above and herein in combination with a clinical symptom score, an epithelial score, and/or a lamina propria ("LP") score.

Also described herein is a clinical symptom score and a process for determining the same by:
(a) determining if the individual exhibits a symptom in any one or more of the following conditions category or categories: 1) heartburn; 2) abdominal pain; 3) nocturnal awakening with symptoms; 4) nausea, regurgitation or vomiting; 5) anorexia or early satiety; or 6) dysphagia, odynophagia, or food impaction;
(b) scoring each condition category (e.g., in one non-limiting example: on a 0 to 3 scale, wherein 0 = individual exhibits none of the symptoms in the condition category; 1 = symptoms limited to 1-3 days or no symptoms in the condition category because coping behaviors are used by the individual; 2 = individual exhibits symptoms for greater than 3 days, with or without minor coping behaviors; and 3 = symptoms in the condition category interfered with activities of daily living or symptoms persisted and required major coping behaviors); and
(c) if more than one condition category is scored, optionally determining a total clinical symptom score by adding the score for each condition category, if more than one category is assessed.

Determination and/or scoring of any process herein (e.g., symptom scoring) can be determined in any suitable manner, e.g., by the patient/subject (e.g., by use of a diary for symptom scoring), care provider, or investigator. Scoring comparisons may also be conducted in any suitable manner. For example, scoring and/or efficacy (e.g., response) determinations may be determined by comparing final scores to initial scores. Alternatively, scoring and/or efficacy determinations may be determined by comparing scoring averages or total scores over the course of a few days, a week, multiple weeks, months, or the like (e.g., wherein the scores are obtained daily, weekly, etc.). If an efficacy determination is being made, such scores may be compared to an earlier scoring average or total score, such as one over the same period of time (e.g., a score determined prior to or at the outset of therapy).

In some embodiments, provided herein is a process of diagnosing eosinophilic esophagitis, measuring response to therapy in an individual with eosinophilic esophagitis, or determining reduction of eosinophilic infiltration by visual appearance, such as by (e.g., an endoscopy score is determined by):
(a) visually inspecting one or more sites of the esophagus, e.g., for one or more of the following conditions 1) pallor and diminished vascular markings; 2) furrowing with thickened mucosa; 3) presence of white mucosal plaques; and 4) concentric rings or strictures;
(b) scoring each inspected condition (e.g., a non-limiting example includes using a 0 to 2 scale, wherein 0 = the condition is not present, 1= the condition is present in 1 or 2 esophageal sites, and 2 = the condition is present in all three esophageal sites, and the individual has an endoscopy score of up to 8).

In some embodiments, reduction of such visual scoring indicates a reduction in eosinophilic infiltration of the esophagus, eosinophilic esophagitis, or symptoms thereof. In certain embodiments, such therapies provide a visual or endoscopy score reduced by at least 25%, at least 50%, at least 75% or the like compared to pretreatment score.

In some embodiments, eosinophilic infiltration of the esophagus is reduced when the individual exhibits a clinical symptom score, as determined above, of a predetermined value, *e*.*g*., in the symptom score method described directly above, or in reduction of score as compared to the score at, or before, initiation of therapy as described in the various embodiments of the invention described herein.

In some embodiments of the invention, reduction is achieved when there is a 50% reduction in score. In other embodiments, there is a 5% reduction in score, a 10% reduction in score, a 15% reduction in score, a 20% reduction in score, a 25% reduction in score, a 30% reduction in score, a 35% reduction in score, a 40% reduction in score, a 45% reduction in score, a 55% reduction in score, a 60% reduction in score, a 65% reduction in score, a 70% reduction in score, a 75% reduction in score, an 80% reduction in score, an 85% reduction in score, a 90% reduction in score, a 95% reduction in score, or a 100% reduction in score. In other embodiments, reduction is achieved when a reduction is of a predetermined value.

In some embodiments of the invention, a method according to the present invention will only be utilized if an individual exhibits a clinical symptom score (e.g., according to the disclosure above) above a predetermined value. In some embodiments, such clinical symptom score above a predetermined value characterizes an esophageal inflammatory condition, or, e.g., EE (or EoE).

In some embodiments, the epithelial score can be determined using any suitable method. In one embodiment, the epithelial score is determined as follows: (a) determining by biopsy, whether the individual's esophagus exhibits a condition in one of the following condition categories in the table below; (b) scoring each condition category according to the table below; and (c) determining a total epithelial score by adding the score for each condition category.

| Condition Category | Score | |
|---|---|---|
| Basal Layer Hyperplasia | None | 0 |
| | > 50% total epithelial thickness | 1 |
| | < 50% total epithelial thickness | 2 |
| Prevalence of Hyperplasia | N/A | 0 |
| | < 50% total biopsy volume | 1 |
| | > 50% total biopsy volume | 2 |
| Dilated Intercellular Spaces | Absent | 0 |
| | Present | 1 |
| Prevalence of Dilated Intercellular Spaces | N/A | 0 |
| | < 50% total biopsy volume | 1 |
| | > 50% total biopsy volume | 2 |
| Eos Microabscesses | None | 0 |
| | 1-2 microabscesses | 1 |
| | 3-4 microabscesses | 2 |
| | ≥ 5 microabscesses | 3 |
| Surface Layering | None | 0 |
| | < 50% total biopsy volume | 1 |
| | > 50% total biopsy volume | 2 |

In some embodiments, eosinophilic infiltration of the esophagus is reduced when the individual exhibits an epithelial score, as determined above, of a predetermined value, *e*.*g*., in the epithelial score method described directly above, or in reduction of score as compared to the score at, or before, initiation of therapy as described in the various embodiments of the invention described herein. In some embodiments of the invention, reduction is achieved when there is a 50% reduction in score. In other embodiments, there is a 5% reduction in score, a 10% reduction in score, a 15% reduction in score, a 20% reduction in score, a 25% reduction in score, a 30% reduction in score, a 35% reduction in score, a 40% reduction in score, a 45% reduction in score, a 55% reduction in score, a 60% reduction in score, a 65% reduction in score, a 70% reduction in score, a 75% reduction in score, an 80% reduction in score, an 85% reduction in score, a 90% reduction in score, a 95% reduction in score, or a 100% reduction in score. In other embodiments, reduction is achieved when a reduction is of a predetermined value.

In some embodiments of the invention, a method according to the present invention will only be utilized if an individual exhibits an epithelial score (e.g., according to the disclosure above) above a predetermined value. In some embodiments, such epithelial score above a predetermined value characterizes an esophageal inflammatory condition, or, e.g., EE (or EoE).

In some embodiments, the endoscopy score can be determined using methods known or determined by those of skill in the pertinent art. In one embodiment, endoscopic examination is performed and findings are recorded with respect to four categories:
1) pallor and/or diminished vascular markings;
2) furrowing with thickened mucosa;
3) presence of white mucosal plaques; and
4) concentric rings or strictures,
and an endoscopy score for each category is determined, for example; zero points if no esophageal sites are involved, one point allocated if 1 or 2 esophageal sites are involved, and two points for pan-esophageal involvement, with a maximum endoscopy score of 8. In some embodiments, eosinophilic infiltration of the esophagus is reduced when the individual exhibits an endoscopy score, as calculated above, of a predetermined value, *e*.*g*., in the endoscopy score method described directly above, or in reduction of score as compared to the score at, or before, initiation of therapy as described in the various embodiments of the invention described herein. In some embodiments of the invention, reduction is achieved when there is a 50% reduction in score. In other embodiments, there is a 5% reduction in score, a 10% reduction in score, a 15% reduction in score, a 20% reduction in score, a 25% reduction in score, a 30% reduction in score, a 35% reduction in score, a 40% reduction in score, a 45% reduction in score, a 55% reduction in score, a 60% reduction in score, a 65% reduction in score, a 70% reduction in score, a 75% reduction in score, an 80% reduction in score, an 85% reduction in score, a 90% reduction in score, a 95% reduction in score, or a 100% reduction in score. In other embodiments, reduction is achieved when a reduction is of a predetermined value.

In some embodiments, the LP score can be determined using methods known or determined by those of skill in the pertinent art. In some embodiments of the invention, a method according to the present invention will only be utilized if an individual exhibits an endoscopy score (e.g., according to the disclosure above) above a predetermined value. In some embodiments, such endoscopy score above a predetermined value characterizes an esophageal inflammatory condition, or, e.g., EE (or EoE).

In one embodiment, the LP score is determined as follows: (a) determining, for example by biopsy, whether the individual's esophagus exhibits a condition in one of the following condition categories in the table below; (b) scoring each condition category according to the table below; and (c) determining a total LP score by adding the score for each condition category.

| Condition Category | Score | |
|---|---|---|
| Lamina Propria Fibrosis | None | 0 |
| | Mild | 1 |
| | Moderate | 2 |
| | Marked | 3 |
| Fibrosis Distribution | N/A | 0 |
| | Superficial | 1 |
| | Superficial and Deep | 2 |
| Prevalence of Fibrosis | N/A | 0 |
| | < 50% lamina propria volume | 1 |
| | > 50% lamina propria volume | 2 |

In some embodiments, eosinophilic infiltration of the esophagus is reduced when the individual exhibits an LP score, as calculated above, of a predetermined value, *e*.*g*., in the LP score method described directly above, or in reduction of score as compared to the score at, or before, initiation of therapy as described in the various embodiments of the invention described herein. In some embodiments of the invention, reduction is achieved when there is a 50% reduction in score. In other embodiments, there is a 5% reduction in score, a 10% reduction in score, a 15% reduction in score, a 20% reduction in score, a 25% reduction in score, a 30% reduction in score, a 35% reduction in score, a 40% reduction in score, a 45% reduction in score, a 55% reduction in score, a 60% reduction in score, a 65% reduction in score, a 70% reduction in score, a 75% reduction in score, an 80% reduction in score, an 85% reduction in score, a 90% reduction in score, a 95% reduction in score, or a 100% reduction in score. In other embodiments, reduction is achieved when a reduction is of a predetermined value.

In some embodiments of the invention, a method according to the present invention will only be utilized if an individual exhibits an LP score (e.g., according to the disclosure above) above a predetermined value. In some embodiments, such LP score above a predetermined value characterizes an esophageal inflammatory condition, or, e.g., EE (or EoE).

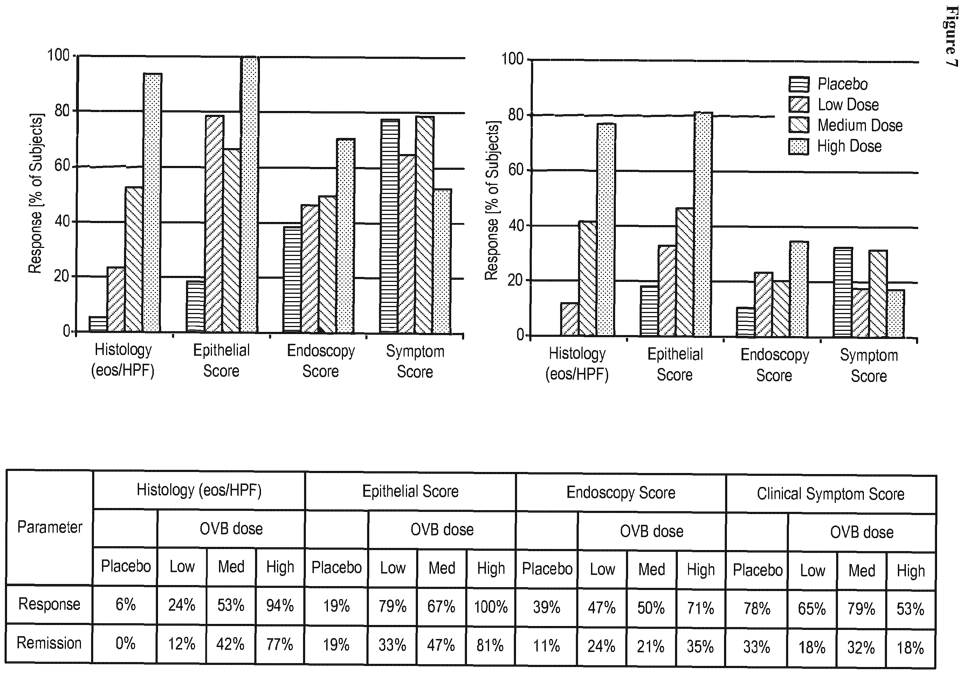

In certain embodiments, provided herein is a method of (1) reducing histology score, (2) reducing epithelial score, (3) reducing endoscopy score, and/or (4) reducing symptom score in an individual suffering from eosinophilic infiltration of the esophagus, the method comprising administering to a subject in need thereof a therapeutically effective amount of corticosteroid (e.g., in a formulation described herein). Figure 7 illustrates therapies described herein whereby such effects are achieved.

In some embodiments, provided herein is a method of or a composition for or suitable for providing one or more of, two or more of, three or more of, four or more of, or all of the following:
a. reducing eosinophilic infiltration of the esophagus (e.g., to ≤ 6 eos/hpf, ≤ 5 eos/hpf, ≤ 4 eos/hpf, ≤ 3 eos/hpf, ≤ 2 eos/hpf, ≤ 1 eos/hpf in one or more or all of the proximal, mid, and distal esophagus);
b. improving the visual appearance of the esophagus (e.g., one or more or all of a reduced endoscopy score, reduced pallor, reduced vascular markings, reduction in furrowing with thickened mucosa, reduction in presence of white mucosal plaques, reduction in concentric rings, and/or reduction in strictures, such as determined by endoscopy);
c. epithelial improvement of the esophagus (e.g., one or more or all of a reduced epithelial score, reduced basal layer hyperplasia, reduced prevalence of hyperplasia, reduced dilated intercellular spaces, reduced prevalence of dilated intercellular spaces, reduced eos microabscesses, and/or reduced surface layering, such as determined by biopsy);
d. reduction of fibrosis of the lamina propia (e.g., one or more or all of a reduced LP score, reduced limina propria fibrosis severity, reduction in fibrosis distribution, and/or reduction in prevalence of fibroses);
e. improvement in symptoms associated with esophageal inflammation (e.g., one or more or all of a reduced symptom score, reduced heartburn, reduced abdominal pain, reduced nocturnal awakening with symptoms, reduced nausea, reduced regurgitation, reduced vomiting, reduced anorexia, reduced early satiety, reduced dysphagia, reduced odynophagia, and/or reduced food impaction).

### Methods for Treating Eosinophilic Esophagitis

The invention also encompasses methods for treating esophageal inflammation in an individual in need thereof, comprising: (a) orally administering to the individual according to a first daily dosing regimen comprising an amount of corticosteroid in an amount effective to reduce eosinophilic infiltration of the esophagus; and subsequently (b) orally administering to the individual according to a second daily dosing regimen comprising an amount of corticosteroid in an amount effective to maintain a reduced level of eosinophilic infiltration of the esophagus.

In one embodiment, the invention encompasses methods for treating eosinophilic esophagitis, the methods comprising:
(a) determining a clinical score (e.g., symptom score, visual score or biopsy score) prior to treatment;
(b) treating the individual for eosinophilic esophagitis by orally administering to the individual any corticosteroid composition described herein (e.g., with a predetermined daily dose of corticosteroid or with an induction dose);
(c) determining a clinical score during treatment; and
(d) optionally adjusting the dose of corticosteroid, e.g., as follows:
   (i) decreasing the dose if the clinical score during treatment is lower than the total clinical score prior to treatment (e.g., with a maintenance dose); and/or
   (ii) increasing the dose if the clinical score during treatment is equal to or higher than the clinical score prior to treatment.In some embodiments, scoring of an induction dose therapy is monitored using a biopsy and/or endoscopy score (optionally in combination with a symptom scoring tool) until a response is determined and a maintenance dose is initiated, and subsequent scoring is monitored utilizing a symptom score.

The invention also encompasses methods for treating esophageal inflammation in an individual in need thereof, comprising: (a) orally administering to the individual according to a first daily dosing regimen comprising an amount of corticosteroid in an amount effective to reduce eosinophilic infiltration of the esophagus; (b) orally administering to the individual according to a second daily dosing regimen comprising an amount of corticosteroid in an amount effective to further reduce eosinophilic infiltration of the esophagus; and subsequently (c) orally administering to the individual according to a third daily dosing regimen comprising an amount of corticosteroid in an amount effective to maintain a reduced level of eosinophilic infiltration of the esophagus.

In specific embodiments therapeutic methods described herein continue for any suitable duration. For example in some embodiments, a subject is administered an induction dose (e.g., for a period of time, such as three months), followed by a maintenance dose (e.g., for 3-12 months). In specific embodiments, the duration of therapy is 3 months, 6 months, 12 months, 18 months, 24 months, or the like. In some instances, a first induction dose is administered to an individual, followed by a second induction dose (e.g., if a full response, such as to less than 6 eosinophils per high field region, or remission, such as to less than 1 eosinophil per high field region) is not achieved in a certain time period (e.g., 1-3 months). In certain embodiments, therapeutic methods described herein comprise administration of a dose for a certain time period (e.g., 3 months), followed by a non-treatment (or off) time period (e.g., 3 months). Following this time on/time off period, the therapy may continue if necessary or desired.

Typically, the corticosteroid is a topically active corticosteroid. Suitable corticosteroids include, but are not limited to, aclometasone, amcinomide, beclometasone, betamethasone, budesonide, ciclesonide, clobetasol, clobetasone, clocortolone, cloprednol, cortivazol, deflazacort, deoxycorticosterone, desonide desoximetasone, dexamethasone, diflorasone, diflucortolone, difluprednate, fluclorolone, fludrocortisone, fludroxycortide, flumetasone, flunisolide, fluocinolone acetonide, fluocinonide, fluocortin, fluocortolone, fluorometholone, fluperolone, fluticasone, fuprednidene, formocortal, halcinonide, halometasone, hydrocortisone aceponate, hydrocortisone buteprate, hydrocortisone butyrate, loteprednol, medrysone, meprednisone, methylprednisolone, methylprednisolone aceponate, mometasone, mometasone furoate, paramethasone, prednicarbate, prednisone, prednisolone, prednylidene, remexolone, tixocortol, triamcinolone, ulobetasol, or a pharmaceutically acceptable salt or ester thereof, or a combination thereof. In some embodiments, the corticosteroid is budesonide, fluticasone, mometasone, desonide, ciclesonide, triamcinolone, beclomethasone, or a pharmaceutically acceptable ester thereof, or a combination thereof. In other embodiments, the corticosteroid is budesonide.

Typically, the individual is a mammal, and, preferably, a human.

In some embodiments, the individual is a child. In other embodiments, the individual is 18 years old or younger. In other embodiments, the individual is less than 17, less than 16, less than 15, less than 14, less than 13, less than 12, less than 11, less than 10, less than 9, less than 8, less than 7, less than 6, less than 5, less than 4, less than 3, less than 2, or less than 1 year old. In certain embodiments, the individual is 12-17 years old, 12-16 years old, or 12-50 years old.

In other embodiments, the individual is 2-9 years old; in other embodiments, the individual is 10-18 years old; in other embodiments, the individual is less than 10 years old; in other embodiments, the individual is 10 or more years old. In other embodiments, the individual is 11 or more, 12 or more, 13 or more, 14 or more, 15 or more, 16 or more, or 18 or more years old. In other embodiments, the individual is 2-5 years old, 6-11 years old, or 12-18 years old.

In some embodiments, the individual is an adult. In other embodiments, the individual is 18 or more years old. In other embodiments, the individual is 21 or more years old, 25 or more years old, 30 or more years old, 35 or more years old, 40 or more years old, 45 or more years old, 50 or more years old, 55 or more years old, 60 or more years old, 65 or more years old, 70 or more years old, 75 or more years old, or 80 or more years old.

### Pharmaceutical compositions

Typically, the corticosteroid is administered to the individual in the form of a pharmaceutical composition. The pharmaceutical composition may comprise a corticosteroid and at least one pharmaceutically acceptable excipient. In some embodiments, the compositions described herein are suitable for oral administration and/or are administered orally, in a manner that delivers the composition or an active therapeutic agent contained therein to a gastrointestinal surface, *e*.*g*., at least a portion of the esophagus.

In one embodiment, the pharmaceutical composition is a liquid pharmaceutical composition. In another embodiment, the pharmaceutical composition is a liquid pharmaceutical composition for oral administration. Suitable liquid pharmaceutical compositions include, but are not limited to, solutions, suspensions, emulsions, syrups, slurries, dispersions, elixirs, and tonics. In other embodiments, the pharmaceutical composition is in the form of a solid oral dosage form. For example, the pharmaceutical composition may also be in the form of an orally dissolving tablet or lozenge. In some embodiments, the composition is in the form of a powder, granules, micropellets, nanopellets, microparticles, nanoparticles, a tablet, an effervescent tablet, a melting tablet, a disintegrating tablet, an orally disintegrating tablet, a foam, a gel, a solid solution, a solid formulation, a solid disintegrating formulation, an emulsion, a liquid or semi-liquid solution, a gum, a wafer (*e*.*g*., dissolving or disintegrating), or a combination thereof. In certain embodiments, the composition is in the form of a film, a patch, a lozenge, or the like. Suitable pharmaceutical compositions are described, for example, in U.S. Publication Nos. 2007/0111978 and 2009/0123550, the contents of which are herein incorporated by reference in their entirety.

Certain embodiments herein provide for a pharmaceutical composition comprising, and in certain embodiments a physically and chemically stable composition comprising,:
a therapeutically effective amount of corticosteroid,
a preservative,
an antioxidant,
a buffer,
a surface active agent or a surfactant,
an optional preservative,
an optional flavoring agent,
an optional sweetener,
at least one additional excipient, and
a liquid vehicle.

In certain embodiments, pharmaceutical preparations for oral use are obtained by combining the corticosteroids with a solid excipient. I'm some instances, such preparations are prepared by, optionally grinding a resulting mixture, and processing the mixture of granules, after adding suitable auxiliaries, if desired, to obtain tablets or dragee cores. Suitable excipients include, by way of non-limiting example, fillers such as sugars or starches, including dextrose, lactose, maltodextrin, sucrose, sucralose, mannitol, or sorbitol; cellulose preparations, for example, maize starch, wheat starch, rice starch, potato starch; other excipients described herein or a combination thereof. Disintegrating agents are optionally added, such as the cross-linked polyvinylpyrrolidone, agar, or alginic acid or a salt thereof such as sodium alginate. In some embodiments, the pharmaceutical compositions used herein include excipients suitable for rendering the dissolving or disintegrating tablet palatable, such as sweeteners or flavoring agents.

In some embodiments, pharmaceutical compositions disclosed herein or used herein comprise a corticosteroid (e.g., budesonide), sucralfate, and, optionally, one or more additional excipient.

In certain embodiments, pharmaceutical compositions disclosed or used herein comprise one or more excipients and/or one or more additional active agents. Excipients useful herein include, by way of non-limiting example, mucoadhesive agents, viscosity enhancing agents, binders, fillers (e.g., corn starch), lubricants, solvents, suspension agents, flavoring agents, coloring agents, sweeteners, preservatives, antioxidants, buffering agents, humectants, chelating agents, surfactants, and the like. As used herein, a mucoadhesive agent is an agent that adheres to a gastrointestinal surface (e.g., either or both of a gastrointestinal epithelia or mucosa).

Additional excipients are as described herein and are used in any suitable amounts, e.g., as described herein. Antioxidants, buffering agents, and surfactants are used in suitable amounts. In certain embodiments, the pharmaceutical composition provided herein is stable. In specific embodiments, the pharmaceutical composition is chemically and/or physically stable.

Preservatives include, by way of non-limiting example, benzalkonium chloride, cetrimide (cetyltrimethylammonium bromide), benzoic acid, benzyl alcohol, methyl-, ethyl-, propyl- and butyl-esters of para-hydroxybenzoic acid, chlorhexidine, chlorobutanol, phenylmercuric acetate, borate and nitrate, potassium sorbate, sodium benzoate, sorbic acid, thiomersal (mercurithiosalicylate), combinations thereof, or the like. Compositions and formulations described herein optionally include about 0.1% w/w to about 5% w/w, about 0.1% w/w to about 3% w/w, about 0.1% w/w to about 1% w/w, about 0.1% w/w to about 0.5% w/w, about 0.2% w/w of one or more preservative(s).

Antioxidants include, by way of non-limiting example, ascorbyl palmitate, butylated hydroxyanisole, butylated hydroxytoluene, monothioglycerol, sodium ascorbate, sodium formaldehyde sulfoxylate, sodium metabisulfite, BHT, BHA, sodium bisulfite, vitamin E or a derivative thereof, propyl gallate, edetate (EDTA) (e.g., disodium edetate), Diethylenetriaminepentaacetic acid (DTPA), Triglycollamate (NT), combinations thereof, or the like. Compositions and formulations described herein optionally include of about 0.01% w/w to about 1% w/w, about 0.01% w/w to about 0.5% w/w, about 0.01% w/w to about 0.3% w/w, or about 0.01% w/w to about 0.1% w/w one or more antioxidant(s).

Buffering agents include, by way of non-limiting example, citrate buffers (i.e., citric acid and citrate), carbonates (e.g., calcium carbonate), hydroxides (e.g., magnesium hydroxide), phosphate buffers, acetate buffers, combinations thereof, or the like.

Humectants are optionally included and may include, by way of non-limiting example, glycerine, propylene glycol, ethylene glycol, glyceryl triacetate, polyols (e.g., sorbitol, xylitol, maltitol, polydextrose), and the like. Compositions and formulations described herein optionally include about 0.1% w/w to about 10% w/w, about 1% w/w to about 10% w/w, about 1% to about 8% w/w, or about 5% w/w of a humectant. In certain embodiments, humectants inhibit or reduce precipitation and/or crystallization of one or more component of a composition or formulation described herein (e.g., a sweetener, mucoadhesive agent or a viscosity enhancing agent).

Chelating agents include, by way of non-limiting example, edetate (EDTA) (e.g., disodium edetate), Diethylenetriaminepentaacetic acid (DTPA), Triglycollamate (NT), or the like. Compositions and formulations described herein optionally include about 0.01% w/w to about 0.5% w/w, about 0.01% w/w to about 0.3% w/w, about 0.01% w/w to about 0.1% w/w, or about 0.05% w/w of one or more chelating agent. In some embodiments, antioxidants or chelating agents (e.g., EDTA) are present in an amount of about 0.05 mg/mL to about 25 mg/mL.

In certain embodiments, sweeteners include, by way of non-limiting example, sugar, glycerin, acesulfame potassium (AceK), mono-ammonium glycyrrhizinate (e.g., Magnasweet^{®}), sucrose, lactose, glucose, fructose, arabinose, xylose, ribose, mannose, galactose, dextrose, sorbose, sorbitol, mannitol, maltose, cellobiose, xylitol and the like. In specific embodiments, the sweetener includes glycerin, acesulfame potassium and mono-ammonium glycyrrhizinate. Sweeteners are optionally included in any suitable amount including, by way of non-limiting example, about 0.01% w/w to about 30% w/w, about 0.1% w/w to about 5% w/w, about 5% w/w to about 20% w/w, about 0.5% w/w, about 0.8% w/w, about 1% w/w, about 6% w/w, about 7% w/w, about 8% w/w, about 9% w/w, about 10% w/w, about 11% w/w, about 12% w/w, about 13% w/w, about 14% w/w, about 15% w/w, about 16% w/w, about 17% w/w, about 18% w/w or about 19% w/w. In some embodiments, flavoring agents include, by way of non-limiting example, peppermint, orange, bubble gum, wintergreen, grape and cherry. Any suitable amount of flavoring agent is optionally utilized including, e.g., about 0.01% w/w, about 0.1% w/w, about 0.2% w/w, about 0.3% w/w, about 0.4% w/w, about 0.5% w/w, about 0.6% w/w, about 0.7% w/w, about 0.8% w/w, about 0.9% w/w, about 1% w/w, up to 5% w/w, up to 10% w/w, or up to 50% w/w. In certain embodiments, a composition described herein has a reduced amount of sugar sweetener (e.g., less than 20% w/w, less than 15% w/w, less than 10% w/w, less than 9% w/w, less than 8% w/w, less than 7% w/w, less than 6% w/w, less than 5% w/w, less than 4% w/w, less than 3% w/w, or less than 2% w/w) and/or a preservative to ensure stability of the composition (e.g., to reduce microbe proliferation). In specific embodiments, glycyrrhizinate such as mono-ammonium glycyrrhizinate (e.g., Magnasweet^{®}) is present in an amount of about 0.01% w/w to about 2.95% w/w. In certain embodiments, coloring agents include yellow agents (e.g., FD&C 5 and/or 6), red agents (e.g., FD&C Red 40, Red No. 3), blue, or the like.

Surfactants include, e.g., anionic, cationic, non-ionic, or zwitterionic surfactants, such as, by way of non-limiting example, polysorbate (e.g., polysorbate 20, polysorbate 60, polysorbate 40, polysorbate 80, polysorbate 81, polysorbate 85, polysorbate 120), bile acids or their salts (e.g., sodium taurocholates, sodium deoxytaurocholates, chenodeoxycholic acid, and ursodeoxycholic acid), nonoxynol or polyoxyethylene glycol fatty acid esters, pluronic or poloxamers such as Pluronic F68, Pluronic L44, Pluronic L101, combinations thereof, or the like. In specific embodiments, the surfactant is polysorbate 80. Compositions and formulations described herein optionally include any suitable amount of surfactant, e.g., about 0.0001% w/w to about 0.5% w/w, about 0.001% w/w to about 0.5% w/w, about 0.001% w/w to about 0.3% w/w, about 0.0005% w/w to about 0.01% w/w, about 0.001% w/w to about 0.1% w/w, about 0.002% w/w, or about 0.01% w/w of one or more surfactant.

In certain embodiments, the additional excipient is selected from, by way of non-limiting example, cellulose (including derivatives thereof), one or more maltodextrin, dextrose, hydroxyethylcellulose (HEC), hydroxypropylmethyl-cellulose (HPMC), carboxymethyl-cellulose (CMC), microcrystalline cellulose (MCC), carbomer and combinations thereof. In more specific embodiments, the at least one additional excipient comprises hydroxyethylcellulose (HEC), hydroxypropylmethyl-cellulose (HPMC), carboxymethyl-cellulose (CMC), microcrystalline cellulose (MCC), carbomer and combinations thereof, and the at least one additional excipient is present in the pharmaceutical composition in an amount of about 5 mg/mL to about 100 mg/mL. In some specific embodiments, the at least one additional excipient comprises, by way of non-limiting example, maltodextrin, dextrose and combinations thereof, and the at least one additional excipient is present in an amount of about 1 mg/mL to about 1.5 g/mL. In some embodiments, the at least one additional excipient comprises CMC, and the CMC is present in the pharmaceutical composition in an amount of about 5 mg/mL to about 30 mg/mL. In certain embodiments, the at least one additional excipient comprises carbomer, and the carbomer is present in the pharmaceutical composition in an amount of about 5 mg/mL to about 100 mg/mL. In some embodiments, the at least one additional excipient comprises HPMC, and the HPMC is present in the pharmaceutical composition in an amount of about 5 mg/mL to about 30 mg/mL. In certain embodiments, the at least one additional excipient comprises MCC, and the MCC is present in the pharmaceutical composition in an amount of about 5 mg/mL to about 30 mg/mL. In some embodiments, the at least one additional excipient comprises a combination of CMC and MCC, and the CMC-MCC combination is present in an amount of about 5 mg/mL to about 40 mg/mL, and wherein the CMC/MCC mixed weight ratio is about 11/89. In certain embodiments, the at least one additional agent comprises dextrose and the dextrose is present in the pharmaceutical composition in an amount of about 10 mg/mL to about 1 g/mL. In some embodiments, the at least one additional agent comprises maltodextrin and the maltodextrin is present in the pharmaceutical composition in an amount of about 10 mg/mL to about 1 g/mL. Other excipients are optionally utilized in formulations described herein; for example, excipients described in US Patent Application Publication No. 2009/0123550 or 2009/0137540, which are incorporated herein by reference for excipients and other components and component amounts described therein.

In one embodiment, the pharmaceutical composition comprises: a therapeutically effective amount of corticosteroid, edetate, citrate, polysorbate 80, an optional preservative, an optional flavoring agent, an optional sweetener, at least one additional excipient, and a liquid vehicle. In some embodiments, the at least one additional excipient increases the interaction of the composition with a gastrointestinal surface, *e*.*g*., the esophagus.

In some embodiments, the at least one additional active agent utilized in a composition, formulation or method described herein is an agent that treats, prevents, or alleviates the symptoms of and/or inflammation associated with inflammatory diseases involving the gastrointestinal tract (e.g., esophagus). In some embodiments, such additional agents provided additive or synergistic effects. In certain embodiments, the at least one additional active agent is an acid inhibitor (e.g., an H2 antagonist and/or a PPI). In certain embodiments, the at least one additional active agent is, by way of non-limiting example, a proton pump inhibitor (PPI), a histamine (e.g., HI, H2, and/or H3) receptor ligand (e.g., antagonist), a transient lower esophageal sphincter relaxation (TLESR)-reducing agent, a prokinetic serotonergic agent, a potassium-competitive acid blocker (P-CAB), a mucosal protectant, an anti-gastrin agent, a leukotriene antagonist, a mast cell inhibitor, a mast cell stabilizer, an immunomodulator, a biologic, an anti-asthmatic agent, a non-steroidal anti-inflammatory drug (NSAID), a chemotherapeutic, mGluR ₅ antagonists, acetylcholine modulator, 5HT ₄ receptor agonist, 5HT ₃ receptor antagonist, 5HT ₁ receptor antagonist, antibiotics, or a combination thereof. In certain embodiments, the at least one additional active agent is an antacid (e.g., calcium carbonate and/or magnesium hydroxide).

PPIs useful herein include, by way of non-limiting example, omeprazole, hydroxyomeprazole, esomeprazole, tenatoprazole, lansoprazole, pantoprazole, rabeprazole, dontoprazole, habeprazole, perprazole, ransoprazole, pariprazole, leminoprazole, S-tenatoprazole-Na, and dexlansoprazole.

In some embodiments, the at least one additional active agent is an H2 receptor ligand (e.g., H2 receptor antagonist). In certain embodiments, H2 receptor antagonists useful herein include, by way of non-limiting example, cimetidine, ranitidine, famotidine and nizatidine. In some embodiments, the therapeutic agent is a H3 receptor ligand (e.g., agonist or antagonist). In certain embodiments, suitable H3 receptor agonists include, by way of non-limiting example, (R)-α-methyl-histamine. In some embodiments, the therapeutic agent is a H1 receptor ligand (e.g., antagonist).

In certain embodiments, the at least one additional active agent is a TLESR-reducing agent. Suitable TLESR-reducing agents include, by way of non-limiting example, GABAB agonists (e.g., baclofen), cholecystokinin (CCK-A or CCK-1) antagonists, anticholinergic agents, NO synthase inhibitors, and combinations thereof.

In some embodiments, the at least one additional active agent is a prokinetic serotonergic agent. In certain embodiments, suitable prokinetic serotonergic agents include, by way of non-limiting example, 5-HT ₄ receptor agonists (e.g., selective 5-HT ₄ receptor agonists). 5-HT ₄ receptor agonists include, by way of non-limiting example, cisapride, mosapride, tegaserod, and ATI-7505.

In some embodiments, the at least one additional active agent is a potassium competitive acid blocker (P-CAB). In certain embodiments, suitable P-CAB include, by way of non-limiting example, soraprazan (BY359), revaprazan (YH1885), AZD0865, CS-526 and combinations thereof.

In certain embodiments, the at least one additional active agent is a mucosal protectant. In some embodiments, suitable mucosal protectants include, by way of non-limiting example, sucralfate. In some embodiments, mucosal protectants include one or more of prostaglandin E ₂ (PGE ₂), epidermal growth factor (EGF) and/or transforming growth factor-a (TGF-α), or analogs thereof. In a specific embodiment, the mucosal protectant comprises the PGE ₂ analog trimoprostil.

In certain embodiments, the at least one additional active agent is an anti-gastrin agent. In some embodiments, suitable anti-gastrin agents include, by way of non-limiting example, cholecystokinin (CCK-B or CCK-2) antagonists. Cholecystokinin (CCK-B or CCK-2) antagonists include, by way of non-limiting example, Z-360.

In some embodiments, the therapeutic agent is a wound healing agent, an agent that promotes cell survival, an agent that promotes cell proliferation, an antifungal agent, an antibacterial agent, an antibiotic, or a combination thereof.

In further embodiments, the at least one additional active agent is a promotility agent. In some embodiments, promotility agents include, by way of non-limiting embodiments, metoclopramide, cisapride, bethanechol, or the like.

In some embodiments, the at least one additional active agent is a chemotherapeutic agent. In some embodiments, chemotherapeutic agents include, by way of non-limiting example, 5-fluorouracil, cisplatin, docetaxel, irinotecan, mitomycin, paclitaxel, vindesine, vinorelbine, and the like.

In certain embodiments, the at least one additional active agent is a mast cell inhibitor. In some embodiments, suitable mast cell inhibitors include, by way of non-limiting example, cromolyn, cromolyn sodium, nedocromil, and the like. In certain embodiments, the therapeutic agent is a leukotriene antagonist. In some embodiments, suitable leukotriene antagonists include, by way of non-limiting example, montelukast, zafirlukast, zileuton, and the like. In some embodiments, mast cell inhibitors described herein (e.g., montelukast) are present in a composition or dose of a composition described herein in an amount sufficient to provide to an individual about 0.1 to about 20 mg/day, about 0.3 to about 4 mg/day, about 10 mg/day to about 500 mg/day, about 20 mg/day to about 40 mg/day, about 20 mg/day to about 100 mg/day, or any other therapeutically effective amount.

In some embodiments, the at least one additional active agent is a non-steroidal anti-inflammatory drug (NSAID). In specific embodiments, the NSAID is ketoprofen. In various other embodiments, the therapeutic agent is an anti-inflammatory agent, e.g., one as set forth in WO 2008/070129, which reference is hereby incorporated by reference in its entirety.

In some embodiments, the at least one additional active agent is an immunomodulator or immunosuppressant. In specific embodiments, the immunomodulator is 6-mercaptopurine (6 MP), azathioprine, suplatast tosylate, mycophenolate mofetil, lactobacillus, calcineurin inhibitors (e.g., tacrolimus, cyclosporine, or the like), or the like.

In certain embodiments, the at least one additional active agent is a biologic. In specific embodiments, the biologic is an anti IL5, an anti TNF (e.g., TNF-α), an IFN (e.g., IFN-α), an anti-eotaxin-1 antibody, an anti IL-3, an anti IgE, omalizumab, reslizumab, mepolizumab, daclizumab, SCH55700, or the like.

In some embodiments, chemotherapeutic agents include, by way of non-limiting example, imatinib, imatinib mesylate, dasatinib, AMN107, cladribine, or the like.

In some embodiments, the at least one additional active agent is an antispasmodic, an agent for treating chest pain (e.g., nitrates, nitroglycerine, or the like), a drug normally administered sublingually (e.g., vitamins, minerals, or the like), mepoliz, esomepraz, STI571, imatinib, an anti-CD25 (e.g., daclizumab), tyrosine kinase inhibitors, somatostatin, somatostatin analogs, an anti-CCR-3, an anti-TIM-3, ketotifen, a platelet derived growth factor receptor (PDGFR) inhibitor, or the like.

The corticosteroid is administered to the individual in an amount effective to achieve its intended purpose, for example, reducing eosinophilic infiltration of the esophagus, including inducing remission of eosinophilic infiltration of the esophagus, or treating esophageal inflammation. In certain embodiments, the exact dosage of corticosteroid depends upon, by way of non-limiting example, the form in which the composition is administered, the subject to be treated, the age, body weight and/or height of the subject to be treated, the preference and experience of the attending physician, the specific corticosteroid used, the characteristics of the patient, and/or the nature of the inflammation for which the treatment is sought. Thus, in some embodiments, the dosage of corticosteroid administered may vary from those disclosed herein. In various embodiments, these factors are determined by those of skill in the medical and pharmaceutical arts in view of the present disclosure.

In various embodiments, the amount of corticosteroid used in a method or in a composition described herein is, for example, from about 2.5-400 µg/kg of body weight per day, about 5-300 µg/kg per day, about 5-200 µg/kg per day, about 5-100 µg/kg per day, about 10-100 µg/kg per day, about 10-50 µg/kg per day, about 10-100 µg/kg/day, or about 5-50 µg/kg/day. In one illustrative embodiment, the amount of corticosteroid used in a method or in a composition described herein is about 10-60 µg/kg/day.

In some embodiments, the corticosteroid is administered in an amount of about 0.01-20 mg per day. In other embodiments, the corticosteroid is administered in an amount of about 0.035 mg or more, or 0.25 mg or more per day. In other embodiments, the corticosteroid is administered in an amount of about 0.5 mg or more per day. In other embodiments, the corticosteroid is administered in an amount of about 0.01-10 mg, about 0.01-5 mg, about 0.01-1 mg, about 0.035-20 mg, about

0.035-5 mg, about 0.035-1 mg, about 0.35-20 mg, about 0.35-10 mg, about 0.35-5 mg, about 0.35-1 mg, about 0.5-20 mg, about 0.1-20 mg, about 0.5 mg-10 mg, about 0.5-6 mg, about 0.5-5 mg, about 0.5-4 mg, about 0.5-3 mg, about 0.5-2 mg, about 1-6 mg, about 1-5 mg, about 1-4 mg, about 1-3 mg, about 1-2 mg, about 2-3 mg, about 2-4 mg, or about 3-4 mg per day. In other embodiments, the corticosteroid is administered in an amount of about 0.035, 0.35 mg, 0.5 mg, 1.4 mg, 1.5 mg, 2 mg, 3 mg, 4 mg, 5 mg, 6 mg, 7 mg, 8 mg, 9 mg, 10 mg, 11 mg, 12 mg, 13 mg, 14 mg, or 15 mg per day.

Provided in certain embodiments herein are methods for reducing, including inducing remission of, eosinophilic infiltration of the esophagus in an individual in need thereof, comprising orally administering to the individual an effective amount of a corticosteroid, wherein the eosinophilic infiltration responds in a dose responsive manner. In some embodiments, provided herein is a method wherein the amount of corticosteroid administered is adjusted to balance efficacy of the therapy with side effects of the therapy.

In an illustrative embodiment, a dosage or amount (including a divided dose) of corticosteroid is provided in a composition of sufficient volume to allow any of the compositions disclosed herein to reach the targeted and/or inflamed portion of the esophagus, in an effective amount. In some embodiments, the effective amount of the composition delivered to the esophagus is an amount sufficient to coat or at least partially coat the esophagus. In certain embodiments, a composition described herein as a volume of, for example, about 1-20 mL, about 1-50 mL, about 1-40 mL, about 1-30 mL, about 1-25 mL, about 5-25 mL, about 10-20 mL, about 7 mL, about 10 mL, about 15 mL, about 20 mL, about 1-15 mL, about 1-10 mL, about 2-8 mL, about 3-7 mL, about 4-6 mL, about 5 mL, about 6-14 mL, about 8-12 mL, or about 9-11 mL.

In more specific embodiments, about 0.01 to about 20 mg, about 0.035 mg to about 20 mg, about 0.035 to about 0.5 mg, about 0.035 to about 1 mg, about 0.05 mg to about 20 mg, about 0.05 mg to about 10 mg, about 0.1 mg to about 10 mg, 0.25 mg to about 6 mg, about 0.25 mg, about 0.375 mg, about 0.5 mg, about 0.75 mg, about 1 mg, about 1.25 mg, about 1.5 mg, about 2 mg, about 3 mg, about 4 mg, about 5 mg, about 6 mg, about 7 mg, about 8 mg, about 9 mg, about 10 mg, about 11 mg, about 12 mg, about 13 mg, about 14 mg, or about 15 mg of corticosteroid is formulated into a single or unit dose of a pharmaceutical composition described herein, the single or unit dose having a total volume of about 1-20 mL, or about 10-20 mL, or for example about 1-15 mL, or for example about 1-10 mL, or for example about 2-10 mL, or for example about 2-15 mL, or for example about 3-15 mL, or for example about 4-15 mL, or for example about 5-15 mL, or for example less than about 1 mL, or for example about 2-8 mL, or for example about 3-7 mL, or for example, about 4-6 mL, or for example, about 5 mL, or for example, about 5-7 mL, or for example about 6-14 mL, or for example about 8-12 mL, or for example, about 9-11 mL, or for example, about 1.5 mL, or for example, about 2 mL, or for example, about 3 mL, or for example, about 4 mL, or for example, about 5 mL, or for example, about 6 mL, or for example, about 7mL, or for example, about 8 mL, or for example, about 9 mL, or for example, about 10 mL, or for example, about 11 mL, or for example, about 12 mL, or for example, about 13 mL, or for example, about 14 mL, or for example, about 15 mL, or for example, about 16 mL, or for example, about 17 mL, or for example, about 18 mL, or for example, about 19 mL, or for example, about 20 mL, or for example, about 21 mL, or for example, about 22 mL, or for example, about 23 mL, or for example, about 24 mL, or for example, about 25 mL.

In some embodiments, the composition comprises corticosteroid in an amount greater than about 0.01 mg per mL, greater than about 0.035 mg per mL, greater than about 0.05 mg per mL, greater than about 0.07 mg per mL, or greater than about 0.1 mg per mL of total volume of the composition. In other embodiments, the composition comprises corticosteroid in an amount of about 0.05-0.4 mg per mL, about 0.05-0.2 mg per mL, about 0.07-0.2 mg per mL, about 0.1-0.2 mg per mL, about 0.2 mg per mL, about 0.05 mg per mL, about 0.01-5 mg per mL, about 0.01-1 mg per mL, about 0.035-5 mg per mL, about 0.01-10 mg per mL, or about 0.035-1 mg per mL of total volume of the composition.

In some embodiments, an appropriate palatable dosage is in a volume sufficient to coat or at least partially coat the esophagus, and in an illustrative embodiment, the volume is sufficient to coat or at least partially coat the esophagus and deliver the corticosteroid to the affected areas, including by way of example only, the entire esophagus, the upper esophagus, the mid esophagus, the lower esophagus, the upper and mid esophagus, the lower and mid esophagus, the esophageal-stomach juncture, the stomach and/or the duodenum.

In one embodiment, when the individual is 10 or more years old, including in some embodiments 10-18 years old and including in other embodiments adults age 19 years and older, the corticosteroid is administered in a composition having a total volume of about 5-15 mL, about 6-14 mL, about 8-12 mL, about 9-11 mL, or about 10 mL. In another embodiment, when the individual is less than 10 years old, including in some embodiments 2-9 years old the corticosteroid is administered in a composition having a total volume of about 5 -10 mL, about 6-8 mL, or about 7 mL.

The composition may be delivered, for example, four times a day, three times a day, twice a day, once a day, every other day, three times a week, twice a week, once a week, or other appropriate intervals. The dosage may, for example, be divided into multiple doses throughout the day, or be provided, for example, in four, three, two, or one dose a day. In certain embodiments, administration comprises more frequent administration (e.g., b.i.d. versus once a day). In some of these embodiments, more frequent administration can provide for a shorter overall therapy, a quicker onset of symptom resolution, a quicker time to remission, or longer time in remission. In one illustrative example, the dose is provided once a day.

In certain embodiments, a dose or composition described herein is administered with food. In some embodiments, a dose or composition described herein is administered without food. In certain embodiments, a dose or composition described herein is administered in a fed or fasted state. In some embodiments, a dose or composition described herein is administered in the morning, in the afternoon, in the evening, at night, or a combination thereof. In some embodiments, the dose is administered at night. In another aspect, the dose is administered about 30 minutes prior to bed, with no food or water given after administration of the compositions herein. In yet another embodiment of the instant invention, the dose is administered prior to bedtime, wherein after administration of the composition, the patient or individual is in a substantially supine position for at least 30 minutes, at least 1 hour, at least 2 hours, at least 4 hours or at least 8 hours.

In certain embodiments, a dose or composition or administration of corticosteroid (e.g., budesonide) according to a method described herein provides a pharmacokinetic profile that is reduced (e.g., so as to reduce side effects associated with systemic corticosteroid delivery). In some embodiments, the reduced pharmacokinetic profile of plasma corticosteroid levels is reduced as compared to systemic therapies (e.g., oral prednisone) for achieving the treatment results described herein. In certain embodiments, the reduced pharmacokinetic profile of plasma corticosteroid levels refers to the achievement of therapeutic effect without providing any or significant side effects. In some instances, provided herein are methods of reducing eosinophilic infiltration of the esophagus (e.g., inducing remission thereof), or symptoms associated therewith and reducing incidences of systemic exposure to and/or systemic side effects resulting from corticosteroid therapy.

In some embodiments, a dose or composition or administration of corticosteroid (e.g., budesonide) according to a method described herein provides a plasma AUC₀₋₈ₕ of less than 5000 hr^{∗}pg/mL, less than 4500 hr^{∗}pg/mL, less than 4200 hr^{∗}pg/mL, less than 4000 hr^{∗}pg/mL, less than 3800 hr^{∗}pg/mL, less than 3750 hr^{∗}pg/mL, less than 3650 hr^{∗}pg/mL, less than 3500 hr^{∗}pg/mL, less than 3400 hr^{∗}pg/mL, less than 1500 hr^{∗}pg/mL, less than 1300 hr^{∗}pg/mL, less than 1200 hr^{∗}pg/mL, less than 1100 hr^{∗}pg/mL, less than 1000 hr^{∗}pg/mL, less than 900 hr^{∗}pg/mL, 200-1500 hr^{∗}pg/mL, 500-1300 hr^{∗}pg/mL, or the like. In certain embodiments, a dose or composition or administration of corticosteroid (e.g., budesonide) according to a method described herein provides a mean or median plasma AUC₀₋₈ₕ of less than 5000 hr^{∗}pg/mL, less than 4500 hr^{∗}pg/mL, less than 4200 hr^{∗}pg/mL, less than 4000 hr^{∗}pg/mL, less than 3800 hr^{∗}pg/mL, less than 3750 hr^{∗}pg/mL, less than 3650 hr^{∗}pg/mL, less than 3500 hr^{∗}pg/mL, less than 3400 hr^{∗}pg/mL, less than 1500 hr^{∗}pg/mL, less than 1300 hr^{∗}pg/mL, less than 1200 hr^{∗}pg/mL, less than 1100 hr^{∗}pg/mL, less than 1000 hr^{∗}pg/mL, less than 900 hr^{∗}pg/mL, 200-1500 hr^{∗}pg/mL, 500-1300 hr^{∗}pg/mL, or the like.

In some embodiments, a dose or composition or administration of corticosteroid (e.g., budesonide) according to a method described herein provides a plasma Cₘₐₓ of less than 2000 pg/mL, less than 1800 pg/mL, less than 1600 pg/mL, less than 1500 pg/mL, less than 1300 pg/mL, less than 1200 pg/mL, less than 1100 pg/mL, less than 1000 pg/mL, less than 500 pg/mL, less than 450 pg/mL, less than 400 pg/mL, less than 350 pg/mL, 100-2000 pg/mL, 200-1500 pg/mL, 750-1250 pg/mL, or the like. In certain embodiments, a dose or composition or administration of corticosteroid (e.g., budesonide) according to a method described herein provides a mean or median plasma Cₘₐₓ of less than 2000 pg/mL, less than 1800 pg/mL, less than 1600 pg/mL, less than 1500 pg/mL, less than 1300 pg/mL, less than 1200 pg/mL, less than 1100 pg/mL, less than 1000 pg/mL, less than 500 pg/mL, less than 450 pg/mL, less than 400 pg/mL, less than 350 pg/mL, 100-2000 pg/mL, 200-1500 pg/mL, 750-1250 pg/mL, 800-1200 pg/mL, 850-1150 pg/mL, 900-1100 pg/mL, 950-1050 pg/mL, or the like.

In some embodiments, a dose or composition or administration of corticosteroid (e.g., budesonide) according to a method described herein provides a plasma Tₘₐₓ of less than 2 hours, less than 1.5 hours, less than 1.2 hours, less than 1.1 hour, less than 1 hour, less than 0.9 hours, about 0.9 hours, about 1.15 hours, about 1.2 hours, about 1 hour, 0.8-1.4 hours, 0.5-1.5 hours, or the like. In certain embodiments, a dose or composition or administration of corticosteroid (e.g., budesonide) according to a method described herein provides a mean or median plasma Tₘₐₓ of less than 2 hours, less than 1.5 hours, less than 1.2 hours, less than 1.1 hour, less than 1 hour, less than 0.9 hours, about 0.9 hours, about 1.15 hours, about 1.2 hours, about 1 hour, 0.8-1.4 hours, 0.5-1.5 hours, or the like.

In some embodiments, a dose or composition or administration of corticosteroid (e.g., budesonide) according to a method described herein provides a plasma Tₘₐₓ of less than 2 hours, a plasma Cₘₐₓ of less than 2000 pg/mL, and a plasma AUC₀₋₈ₕ of less than 5000 hr^{∗}pg/mL (e.g., wherein such plasma levels are median or mean values, or individual values). In certain embodiments, a dose or composition or administration of corticosteroid (e.g., budesonide) according to a method described herein provides a plasma Tₘₐₓ of less than 1.5 hours, a plasma Cₘₐₓ of less than 1500 pg/mL, and a plasma AUC₀₋₈ₕ of less than 4000 hr^{∗}pg/mL (e.g., wherein such plasma levels are median or mean values, or individual values). In some embodiments, a dose or composition or administration of corticosteroid (e.g., budesonide) according to a method described herein provides a plasma Tₘₐₓ of less than 1.2 hours, a plasma Cₘₐₓ of less than 1200 pg/mL, and a plasma AUC₀₋₈ₕ of less than 36000 hr^{∗}pg/mL (e.g., wherein such plasma levels are median or mean values, or individual values). In some embodiments, a dose or composition or administration of corticosteroid (e.g., budesonide) according to a method described herein provides a median plasma Tₘₐₓ of less than 1.1 hours, a median plasma Cₘₐₓ of less than 850 pg/mL, and a median plasma AUC₀₋₈ₕ of less than 2800 hr^{∗}pg/mL. In certain embodiments, a dose or composition or administration of corticosteroid (e.g., budesonide) according to a method described herein provides a mean plasma Tₘₐₓ of less than 1.2 hours, a mean plasma Cₘₐₓ of less than 1100 pg/mL, and a mean plasma AUC₀₋₈ₕ of less than 3600 hr^{∗}pg/mL.

In some embodiments, a dose or composition or administration of corticosteroid (e.g., budesonide) according to a method described herein provides a plasma T_{1/2} of less than 8 hours, less than 6 hours, less than 5 hours, less than 4 hours, less than 3.8 hours, less than 3.5 hours, less than 3 hours, about 3.3 hours, about 4 hours, about 3 hours, about 3.4 hours, about 3.2 hours, about 3 hours, about 2.5 hours, 2.3-4.5 hours, 2-5 hours, 3-4 hours, or the like. In certain embodiments, a dose or composition or administration of corticosteroid (e.g., budesonide) according to a method described herein provides a mean or median plasma T_{1/2} of less than 8 hours, less than 6 hours, less than 5 hours, less than 4 hours, less than 3.8 hours, less than 3.5 hours, less than 3 hours, about 3.3 hours, about 4 hours, about 3 hours, about 3.4 hours, about 3.2 hours, about 3 hours, about 2.5 hours, 2.3-4.5 hours, 2-5 hours, 3-4 hours, or the like.

Additionally, in some instances, provided herein are methods of reducing eosinophilic infiltration of the esophagus (e.g., inducing remission thereof), or symptoms associated therewith and reducing incidences of local side effects resulting from topical corticosteroid therapy, such as candidiasis (e.g, oral, esophageal, oropharyngeal) or thrush. In specific embodiments, the method comprises adverse candidiasis (e.g, oral, esophageal, oropharyngeal) or thrush in less than 15% of subjects, less than 10% of subjects, less than 8% of subjects, less than 6% of subjects, less than 5% of subjects, less than 4% of subjects, less than 2% of subjects, or the like.

In certain embodiments, provided herein is a method providing the reduction of conditions associated with an esophageal disorder (e.g., esophageal inflammation, such as eosinophilic esophagitis). In certain instances, such conditions are scored according to the following chart:

| Condition Category | Score | |
|---|---|---|
| Basal Layer Hyperplasia | None | 0 |
| | > 50% total epithelial thickness | 1 |
| | < 50% total epithelial thickness | 2 |
| Prevalence of Hyperplasia | N/A | 0 |
| | < 50% total biopsy volume | 1 |
| | > 50% total biopsy volume | 2 |
| Dilated Intercellular Spaces | Absent | 0 |
| | Present | 1 |
| Prevalence of Dilated Intercellular Spaces | N/A | 0 |
| | < 50% total biopsy volume | 1 |
| | > 50% total biopsy volume | 2 |
| Eos Microabscesses | None | 0 |
| | 1-2 microabscesses | 1 |
| | 3-4 microabscesses | 2 |
| | ≥ 5 microabscesses | 3 |
| Surface Layering | None | 0 |
| | < 50% total biopsy volume | 1 |
| | > 50% total biopsy volume | 2 |

In some embodiments, a dose or composition or administration of corticosteroid (e.g., budesonide) according to a method described herein provides (based on the previous scoring system) at least a 3 point reduction, at least a 2 point reduction, or at least a 1 point reduction. In some embodiments, a dose or composition or administration of corticosteroid (e.g., budesonide) according to a method described herein provides (based on the previous scoring system) a mean reduction of at least 5 points, at least 4.5 points, at least 4 points, at least 3.5 points, at least 3 points, at least 2.5 points, at least 2 points, at least 1.5 points, at least 1 point (e.g., wherein the baseline score or mean baseline score is at least 4 points, at least 5 points, about 4.8 points, about 5 points, about 5.1 points, about 5.2 points, or the like). In some embodiments, a dose or composition or administration of corticosteroid (e.g., budesonide) according to a method described herein provides (based on the previous scoring system) at least a 50% point reduction, at least a 60% point reduction, at least a 70% point reduction, at least an 80% point reduction, or at least a 90% point reduction. In some embodiments, a dose or composition or administration of corticosteroid (e.g., budesonide) according to a method described herein provides (based on the previous scoring system) a mean reduction of at least a 95%, at least a 90% points, at least a 85% points, at least 80% points, at least 75% points, at least 70% points, at least 60% points, at least 50% points, or at least 25% point (e.g., wherein the baseline score or mean baseline score is at least 4 points, at least 5 points, about 4.8 points, about 5 points, about 5.1 points, about 5.2 points, or the like). In some embodiments, a dose or composition or administration of corticosteroid (e.g., budesonide) according to a method described herein provides (based on the previous scoring system) a reduction to less than 3 points, less than 2 points, or less than 1 point. In some embodiments, a dose or composition or administration of corticosteroid (e.g., budesonide) according to a method described herein provides (based on the previous scoring system) a provides (based on the previous scoring system) a reduction to a mean of less than 3 points, less than 2.5 points, less than 2 points, less than 1.8 points, less than 1.5 points, less than 1.3 points, less than 1 point, less than 0.8 points, less than 0.5 points, or the like (e.g., wherein the baseline score or mean baseline score is at least 4 points, at least 5 points, about 4.8 points, about 5 points, about 5.1 points, about 5.2 points, or the like). In some embodiments, a dose or composition or administration of corticosteroid (e.g., budesonide) according to a method described herein provides (based on the previous scoring system) a provides a symptom score of 0-1 or 0-2.

In certain embodiments, provided herein is a method of reducing conditions associated with esophageal inflammation (e.g., eosinophilic esophagitis) or fibrosis (e.g., associated with eosinophilic esophagitis). In certain instances, such conditions are scored according to the following chart:

| Condition Category | Score | |
|---|---|---|
| Lamina Propria Fibrosis | None | 0 |
| | Mild | 1 |
| | Moderate | 2 |
| | Marked | 3 |
| Fibrosis Distribution | N/A | 0 |
| | Superficial | 1 |
| | Superficial and Deep | 2 |
| Prevalence of Fibrosis | N/A | 0 |
| | < 50% lamina propria volume | 1 |
| | > 50% lamina propria volume | 2 |

In some embodiments, a dose or composition or administration of corticosteroid (e.g., budesonide) according to a method described herein provides (based on the previous scoring system) at least a 20% point reduction, at least a 30% point reduction, at least a 40% point reduction, at least a 50% point reduction, at least a 60% point reduction, at least a 70% point reduction, at least an 80% point reduction, or at least a 90% point reduction (e.g., mean point reduction). In some embodiments, a dose or composition or administration of corticosteroid (e.g., budesonide) according to a method described herein provides (based on the previous scoring system) a provides a symptom score of 0-1 or 0-2.

In some embodiments, the administration of the corticosteroid according to the methods of the invention does not alter the individual's morning cortisol levels. In one embodiment, the individual's morning cortisol levels during administration of the corticosteroid are in a normal range, as recognized by one of skill in the pertinent art, and can include, for example, about 5-20 µg/dL, about 8-14 µg/dL, or at a level that does not fall below about 5 µg/dL. In another embodiment, the individual's morning cortisol levels during administration of the corticosteroid are above or equal to about 5 µg/dL.

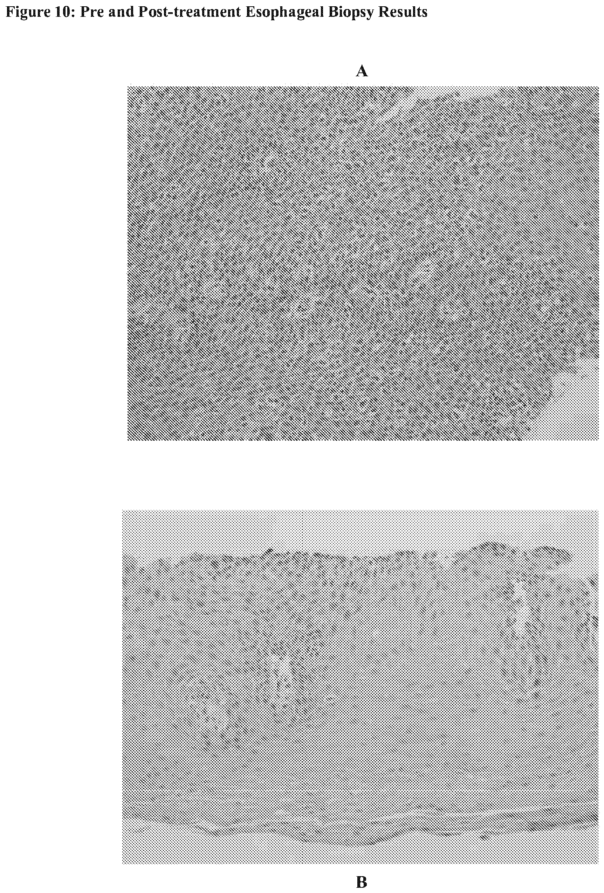

In certain embodiments, the administration of the corticosteroid according to any method described herein provides a reduction of esophageal eosinophil count (e.g., mean, median, or maximum on an individual or average basis) by at least 20%, by at least 30%, by at least 40%, by at least 60%, by at least 70%, by at least 80%, by at least 90%, or by at least 95% after one week of treatment, two weeks of treatment, three weeks of treatment, six weeks of treatment, twelve weeks of treatment, or the like. In some embodiments, the administration of the corticosteroid according to any method described herein provides a reduction (e.g., of maximum, mean, or median) esophageal eosinophil count (on an individual or average basis) by at least 5 eos/hpf, at least 10 eos/hpf, at least 20 eos/hpf, at least 30 eos/hpf, at least 40 eos/hpf, at least 50 eos/hpf, at least 60 eos/hpf, at least 70 eos/hpf, at least 80 eos/hpf, at least 90 eos/hpf, or at least 100 eos/hpf after one week of treatment, two weeks of treatment, three weeks of treatment, six weeks of treatment, twelve weeks of treatment, 3 months, 6 months, 12 months, or the like. In some embodiments, the administration of the corticosteroid according to any method described herein provides a reduction of esophageal eosinophil count (e.g., maximum, mean, or median on an individual or average basis) by at least 10 eos/hpf, at least 20 eos/hpf, at least 30 eos/hpf, at least 40 eos/hpf, at least 50 eos/hpf, at least 60 eos/hpf, at least 70 eos/hpf, at least 80 eos/hpf, at least 90 eos/hpf, or at least 100 eos/hpf after one week of treatment, two weeks of treatment, three weeks of treatment, six weeks of treatment, twelve weeks of treatment, or the like. Figure 10 illustrates the reduction of eosinophils biopsied from an individual treated according to a method described herein (wherein panel A illustrates a biopsy prior to treatment and panel B illustrates a biopsy post treatment).

In some embodiments, the first daily dosing regimen comprising an amount of corticosteroid is less than the second daily dosing regimen comprising an amount of corticosteroid and the third daily dosing regimen comprising an amount of corticosteroid. In some embodiments, the first daily dosing regimen comprising an amount of corticosteroid is less than the second daily dosing regimen comprising an amount of corticosteroid and more than the third daily dosing regimen comprising an amount of corticosteroid. In some embodiments, the first daily dosing regimen comprising an amount of corticosteroid is more than the second daily dosing regimen comprising an amount of corticosteroid and less the third daily dosing regimen comprising an amount of corticosteroid. In other embodiments, the first daily dosing regimen comprising an amount of corticosteroid is more than the second daily dosing regimen comprising an amount of corticosteroid and the third daily dosing regimen comprising an amount of corticosteroid. In some embodiments, the first daily dosing regimen comprising an amount of corticosteroid is less than the second daily dosing regimen comprising an amount of corticosteroid and about the same as the third daily dosing regimen comprising an amount of corticosteroid. In some embodiments, the first daily dosing regimen comprising an amount of corticosteroid is more than the second daily dosing regimen comprising an amount of corticosteroid and about the same as the third daily dosing regimen comprising an amount of corticosteroid. In some embodiments, the first daily dosing regimen comprising an amount of corticosteroid is about the same as the second daily dosing regimen comprising an amount of corticosteroid and less than the third daily dosing regimen comprising an amount of corticosteroid. In some embodiments, the first daily dosing regimen comprising an amount of corticosteroid is about the same as the second daily dosing regimen comprising an amount of corticosteroid and more than the third daily dosing regimen comprising an amount of corticosteroid.

In some embodiments, the eosinophilic infiltration of the esophagus is reduced to an extent sufficient to treat the eosinophilic infiltration in an individual. As used herein, the eosinophilic infiltration is "treated" when the individual's eosinophil count is reduced to 23 or fewer, 15 or fewer, 6 or fewer, or 1 or fewer eosinophils per high power field in one or more sites (*e*.*g*., distal, mid, or proximal) of the esophageal mucosa.

In other embodiments, the eosinophilic infiltration of the esophagus is reduced, and in some embodiments reduced to an extent sufficient to induce remission of the esophageal infiltration. Remission may be defined in any of the manners described above, including by way of non-limiting example, less than 1 eosinophil per high power field in each of the proximal, mid and distal esophageal mucosa. In some instances, a response may be determined by a histological score alone, or a histological score in combination with one or more score, such as an LP score, a symptom score, a visual score, or the like (i.e., the histologic score may indicate a response, or may be one endpoint among two or more endpoints in determining a response - e.g., wherein each endpoint may require a statistically significant result for there to be an affirmative response).

Eosinophil counts are determined, for example, by endoscopy/biopsy. Endoscopy/biopsy can be performed by any method known to one of skill in the art. Esophagogastroduodenoscopy (EGD) with esophageal, gastric and/or duodenal biopsies are techniques known to those of skill in the pertinent art, and are used in certain embodiments of the invention. For example, EGD may be performed by a referring physician prior to a screening visit before utilizing a method of the present invention, or may be performed during a screening period where an individual is being considered for use of a method of the present invention. In some embodiments, EGD is performed prior to the initiation of therapy utilizing a method of the present invention. In some embodiments of the invention, EGD is used for endoscopy alone. In other embodiments, EGD is used for endoscopy and the taking of one or more biopsies. In some embodiments of the invention, an endoscopy with esophageal biopsies is performed during treatment utilizing a method of the present invention. In particular, for all of the above, esophageal biopsies can be used. As described above, biopsies can be taken from the proximal, mid and/or distal area of the esophagus. In some embodiments, biopsies are taken at multiple sites in one area. In one non-limiting example, biopsies are taken at two or, *e*.*g*., 6 sites in each of the proximal, mid and/or distal area of the esophagus.

In some embodiments, endoscopic findings in the esophagus are recorded, and in some embodiments, findings are recorded with respect to four categories:
1) pallor and/or diminished vascular markings;
2) furrowing with thickened mucosa;
3) presence of white mucosal plaques; and
4) concentric rings or strictures.

In some embodiments, an endoscopy score for each category is determined, for example; zero points if no esophageal sites are involved, one point allocated if 1 or 2 esophageal sites are involved, and two points for pan-esophageal involvement, with a maximum endoscopy score of 8.

In some embodiments, esophageal biopsies are performed by an endoscopist within a certain time, *e*.*g*., 6 weeks, prior to utilizing the methods describe herein. In some embodiments where esophageal biopsies are used, multiple specimens (in some embodiments, 2, or at least 2 biopsies from each of 3 levels) are obtained from the proximal (about 3 cm below the crycopharyngeus muscle), distal (about 3 cm above the gastroesophageal junction) and mid esophagus (about mid-point between the crycopharyngeus muscle and the gastroesophageal junction). In some embodiments, biopsies are taken from the stomach and duodenum, for example, as follows: gastric body (greater curvature): 2, gastric antrum: 2, duodenum (third part or distal): 2. In some embodiments if a pre-treatment biopsy identifies disease in the stomach and/or duodenum, the individual will be delayed treatment according to the methods described herein. In some embodiments, a method according to the present invention will only be utilized if one or more pre-treatment biopsies identifies no disease in the stomach and/or duodenum.

In some embodiments, the first daily amount of corticosteroid is an amount effective to reduce the individual's eosinophil count to 23 or fewer, 15 or fewer, 6 or fewer, or 1 or fewer eosinophils per high power field in one or more sites of the esophageal mucosa. In other embodiments, the first daily amount of corticosteroid is an amount effective to reduce the individual's eosinophil count to 23 or fewer, 15 or fewer, 6 or fewer, or 1 or fewer eosinophils per high power field in the distal esophageal mucosa. In other embodiments, the first daily amount of corticosteroid is an amount effective to reduce the individual's eosinophil count to 23 or fewer, 15 or fewer, 6 or fewer, or 1 or fewer eosinophils in the distal esophageal mucosa and the mid esophageal mucosa. In other embodiments, the first daily amount of corticosteroid is an amount effective to reduce the individual's eosinophil count to 23 or fewer, 15 or fewer, 6 or fewer, or 1 or fewer eosinophils in the distal esophageal mucosa, the mid esophageal mucosa, and the proximal esophageal mucosa.

In some embodiments, the first daily amount is about 0.01-20 mg, about 0.01-10 mg, about 0.01-5 mg, about 0.01-1 mg, about 0.035-20 mg, about 0.035-5 mg, about 0.035-1 mg, about 0.35-20 mg, about 0.35-10 mg, about 0.35-5 mg, about 0.35- 1 mg, about 0.5-20 mg, about 0.5 mg-10 mg, about 0.5-6 mg, about 0.5-5 mg, about 0.5-4 mg, about 0.5-3 mg, about 0.5-2 mg, about 1-6 mg, about 1-5 mg, about 1-4 mg, about 1-3 mg, about 1-2 mg, about 2-3 mg, about 2-4 mg, or about 3-4 mg per day. In other embodiments, the first daily amount is about 2 mg or more, about 2-6 mg, about 2-4 mg, about 2-3 mg, or about 3-4 mg per day.

In some embodiments, the first daily amount is administered to the individual for at least about 1 month. In other embodiments, the first daily amount is administered to the individual for about 36 months, about 24 months, about 18 months, about 12 months, about 6 months, about 5 months, about 4 months, about 3 months, about 2 months, about 1 month, about 4 weeks, about 4 weeks to about 6 months, about 4 weeks to about 3 months, about 4 weeks to about 2 months, up to about 2 months, up to about 3 months, up to about 4 months, up to about 5 months, up to about 6 months, about 1-4 weeks, about 1-3 weeks, about 2-3 weeks, about 1-2 weeks, or about 1 week.

In some embodiments, the second daily amount of corticosteroid is administered in an amount effective to maintain the reduction of eosinophilic infiltration achieved by the administration of the first daily amount of corticosteroid.

In some embodiments, the second daily amount of corticosteroid is an amount effective to maintain the individual's eosinophil count at 23 or fewer, 15 or fewer, 6 or fewer, or 1 or fewer eosinophils per high power field in one or more sites of the esophageal mucosa. In other embodiments, the second daily amount of corticosteroid is an amount effective to maintain the individual's eosinophil count at 23 or fewer, 15 or fewer, 6 or fewer, or 1 or fewer eosinophils per high power field in the distal esophageal mucosa. In other embodiments, the second daily amount of corticosteroid is an amount effective to maintain the individual's eosinophil count at 23 or fewer, 15 or fewer, 6 or fewer, or 1 or fewer eosinophils in the distal esophageal mucosa and the mid esophageal mucosa. In other embodiments, the second daily amount of corticosteroid is an amount effective to maintain the individual's eosinophil count at 23 or fewer, 15 or fewer, 6 or fewer, or 1 or fewer eosinophils in the distal esophageal mucosa, the mid esophageal mucosa, and the proximal esophageal mucosa.

In some embodiments, the second daily amount of corticosteroid is administered in an amount effective to further reduce the eosinophilic infiltration achieved by the administration of the first daily amount of corticosteroid. In one embodiment, the second daily amount of corticosteroid is an amount effective to further reduce the individual's eosinophil count to 23 or fewer, to 15 or fewer, 6 or fewer, or 1 or fewer eosinophils per high power field in one or more sites of the esophageal mucosa. In other embodiments, the second daily amount of corticosteroid is an amount effective to further reduce the individual's eosinophil count to 23 or fewer, to 15 or fewer, 6 or fewer, or 1 or fewer eosinophils per high power field in the distal esophageal mucosa. In other embodiments, the second daily amount of corticosteroid is an amount effective to further reduce the individual's eosinophil count to 15 or fewer, 6 or fewer, or 1 or fewer eosinophils in the distal esophageal mucosa and the mid esophageal mucosa. In other embodiments, the second daily amount of corticosteroid is an amount effective to further reduce the individual's eosinophil count to 15 or fewer, 6 or fewer, or 1 or fewer eosinophils in the distal esophageal mucosa, the mid esophageal mucosa, and the proximal esophageal mucosa.

In some embodiments, the second daily amount is about 0.01-20 mg, about 0.01-10 mg, about 0.01-5 mg, about 0.01-1 mg, about 0.035-20 mg, about 0.035-5 mg, about 0.035-1 mg, about 0.35-20 mg, about 0.35-10 mg, about 0.35-5 mg, about 0.35-1 mg, about 0.5-20 mg, about 0.5 mg-10 mg, about 0.5-6 mg, about 0.5-5 mg, about 0.5-4 mg, about 0.5-3 mg, about 0.5-2 mg, about 1-6 mg, about 1-5 mg, about 1-4 mg, about 1-3 mg, about 1-2 mg, about 2-3 mg, about 2-4 mg, or about 3-4 mg per day. In other embodiments, the second daily amount is about 2 mg or less, about 0.5-2 mg, about 1-2 mg, or about 1 mg per day.

In some embodiments, the second daily amount is administered to the individual for at least about 1 month. In other embodiments, the second daily amount is administered to the individual for about 36 months, about 24 months, about 18 months, about 12 months, about 6 months, about 5 months, about 4 months, about 3 months, about 2 months, about 1 month, about 4 weeks, about 4 weeks to about 6 months, about 4 weeks to about 3 months, about 4 weeks to about 2 months, up to about 2 months, up to about 3 months, up to about 4 months, up to about 5 months, up to about 6 months, 5, about 1-4 weeks, about 1-3 weeks, about 2-3 weeks, about 1-2 weeks, about 1 week, or indefinitely.

In some embodiments, the second daily amount is administered until such time as the reduced level of eosinophilic infiltration of the esophagus of step (b) is less than 200%, less than 100%, or less than 50%, less than 25%, or less than 10% of the level of eosinophilic infiltration level achieved in step (a).

In some embodiments, the third daily amount of corticosteroid is administered in an amount effective to maintain the reduction of eosinophilic infiltration achieved by the administration of the second daily amount of corticosteroid. In some embodiments, such reduction includes remission.

In some embodiments, the third daily amount of corticosteroid is an amount effective to maintain the individual's eosinophil count at 15 or fewer, 6 or fewer, or 1 or fewer eosinophils per high power field in one or more sites of the esophageal mucosa. In other embodiments, the third daily amount of corticosteroid is an amount effective to maintain the individual's eosinophil count at 15 or fewer, 6 or fewer, or 1 or fewer esopinophils per high power field in the distal esophageal mucosa. In other embodiments, the third daily amount of corticosteroid is an amount effective to maintain the individual's eosinophil count at 15 or fewer, 6 or fewer, or 1 or fewer eosinophils in the distal esophageal mucosa and the mid esophageal mucosa. In other embodiments, the third daily amount of corticosteroid is an amount effective to maintain the individual's eosinophil count at 15 or fewer, 6 or fewer, or 1 or fewer eosinophils in the distal esophageal mucosa, the mid esophageal mucosa, and the proximal esophageal mucosa.

In some embodiments, the third daily amount is about 0.035-20 mg, about 0.035-0.5 mg, about 0.035-1 mg, about 0.5-20 mg, about 0.5 mg-10 mg, about 0.5-6 mg, about 0.5-5 mg, about 0.5-4 mg, about 0.5-3 mg, about 0.5-2 mg, about 1-6 mg, about 1-5 mg, about 1-4 mg, about 1-3 mg, about 1-2 mg, about 2-3 mg, about 2-4 mg, or about 3-4 mg per day. In other embodiments, the third daily amount is about 2 mg or less, about 0.5-2 mg, about 1-2 mg, or about 1 mg per day.

In some embodiments, the third daily amount is administered to the individual for at least about 1 month. In other embodiments, the third daily amount is administered to the individual for about 6 months, about 5 months, about 4 months, about 3 months, about 2 months, about 1 month, about 4 weeks, about 4 weeks to about 6 months, about 4 weeks to about 3 months, about 4 weeks to about 2 months, up to about 2 months, up to about 3 months, up to about 4 months, up to about 5 months, up to about 6 months, about 1-4 weeks, about 1-3 weeks, about 2-3 weeks, about 1-2 weeks, or about 1 week.

### EXAMPLES

### Example 1: Administration of Oral Viscous Budesonide to Patients with Eosinophilic Esophagitis

82 subjects with eosinophilic esophagitis, 2-18 years of age, having maximum peak eosinophil counts of ≥ 20 per high power field in at least two sites of the esophageal mucosa and a Clinical Symptom Score of ≥ 3 were treated with oral viscous budesonide ("OVB") over a period of 12 weeks. The demographics of the subjects are summarized in Table 1 below:

**Table 1.**

| **Variable** | | **Placebo N=21** | **All OVB (N=60)** | **Total (N=81)** |
|---|---|---|---|---|
| Age (years) | | | | |
| | Mean (SD) | 9.2 (4.4) | 9.1 (5.2) | 9.1 (4.9) |
| | Median | 8.0 | 8.0 | 8.0 |
| | Range | 2, 17 | 1, 18 | 1,18 |
| Gender [n (%)] | | | | |
| | Male | 16 (76.2%) | 50 (83.3%) | 66 (81.5%) |
| | Female | 5 (23.8%) | 10 (16.7%) | 15 (18.5%) |
| Race [n (%)] | | | | |
| | Caucasian | 20 (95.2%) | 57 (95.0%) | 77 (95.1%) |
| | Black | 1 (4.8%) | 2 (3.3%) | 3 (3.7%) |
| | Native | 0 | 1(1.7%) | 1 (1.2%) |
| American | | 0 | 0 | 0 |
| | Asian | | | |
| Height (in) | | | | |
| | n | 20 | 59 | 79 |
| | Mean (SD) | 51.5 (10.2) | 52.9 (11.7) | 52.6 (11.3) |
| Weight (lb) | | | | |
| | n | 20 | 60 | 80 |
| | Mean (SD) | 74.7 (42.7) | 83.4 (52.1) | 81.2 (49.8) |

The subjects were subjected to 4 weeks of screening, followed by 12 weeks of treatment, and then 3 weeks of taper.

20 subjects were given a low dose of OVB (0.35 mg for ages 2-9 and 0.5 mg for ages 10-18) once daily, 20 subjects were given a medium dose of OVB (1.4 mg for ages 2-9 and 2.0 mg for ages 10-18) once daily, 20 subjects were given a high dose (2.8 mg for ages 2-9 and 4 mg for ages 10-18) of OVB divided over two doses daily, and 20 subjects were given placebo over a period of 12 weeks. The dosing regimen is summarized in Table 2 below.

**Table 2.**

| **Dose Group** | **Age Group (Years)** | **Dose Volume [mL]** | **No. Subjects** | **Susp. Cone. (mg/mL)** | **Morning Dose (mg)** | **Evening Dose (mg)** | **Total Dose/Day (mg/day)** |
|---|---|---|---|---|---|---|---|
| Placebo | 2 to 9 | 7 | 20 | 0 | Placebo | Placebo | 0 |
| | 10 to 18 | 10 | | | | | 0 |
| Low | 2 to 9 | 7 | 20 | 0.05 | Placebo | 0.35 | 0.35 |
| | 10 to 18 | 10 | | | | 0.5 | 0.5 |
| Medium | 2 to 9 | 7 | 20 | 0.2 | Placebo | 1.4 | 1.4 |
| | 10 to 18 | 10 | | | | 2.0 | 2 |
| High | 2 to 9 | 7 | 20 | 0.2 | 1.4 | 1.4 | 2.8 |
| | 10 to 18 | 10 | | | 2.0 | 2.0 | 4 |

The formulation of the OVB composition is as follows:

**Table 3.**

| Component | Placebo | | 0.05 mg/mL Strength | | 0.2 mg/mL Strength | |
|---|---|---|---|---|---|---|
| | | | % w/w | mg/mL | % w/w | mg/mL |
| Budesonide | N/A | N/A | 0.004 | 0.05 | 0.017 | 0.2 |
| Microcrystalline cellulose (Avicel RC-591) | 2.0 | 23.60 | 2.0 | 23.60 | 2.0 | 23.60 |
| Maltodextrin M150 | 26.00 | 306.80 | 26.00 | 306.80 | 26.00 | 308.60 |
| Dextrose | 10.00 | 118.00 | 10.00 | 118.00 | 10.00 | 118.00 |
| Disodium edetate | 0.05 | 0.59 | 0.05 | 0.59 | 0.05 | 0.59 |
| Citric acid | 0.15 | 1.77 | 0.15 | 1.77 | 0.15 | 1.77 |
| Sodium citrate | 0.05 | 0.59 | 0.05 | 0.59 | 0.05 | 0.59 |
| Potassium sorbate | 0.20 | 2.36 | 0.20 | 2.36 | 0.20 | 2.36 |
| Polysorbate 80 | 0.01 | 0.12 | 0.01 | 0.12 | 0.01 | 0.12 |
| Glycerine (99%) | 5.0 | 59.00 | 5.0 | 59.00 | 5.0 | 59.00 |
| Sodium benzonate | 0.20 | 2.36 | 0.20 | 2.36 | 0.20 | 2.36 |
| Cherry Flavor | 0.5 | 5.90 | 0.5 | 5.90 | 0.5 | 5.90 |
| Magnasweet 110 | 0.5 | 5.90 | 0.5 | 5.90 | 0.5 | 5.90 |
| Acesulfame potassium | 0.5 | 5.90 | 0.5 | 5.90 | 0.5 | 5.90 |
| Water, purified | 54.84 | q.s. to 1.0 mL | 54.84 | q.s. to 1.0 mL | 54.82 | q.s. to 1.0 mL |
| Total | 100.00 | | 100.00 | | 100.00 | |

### Results:

In these non-limiting examples, treatment groups using a method of the present invention are defined as follows: the Full Analysis Set group includes subjects that had pre- and post-treatment biopsies, where the post-treatment biopsy is taken at about 12 weeks of treatment, or substantially after a majority of weeks of treatment defined in the protocol. The Intent to Treat group includes all subjects who were administered at least one dose of study medication according to a method of the present invention, but did not complete the entire designated protocol, and can include for example, subjects who did not conclude treatment or, in particular, have a post-treatment biopsy. The Per Protocol group (designated at some points herein as "PP") includes subjects that concluded treatment pursuant to the study protocol.

### Histologic Results/Response:

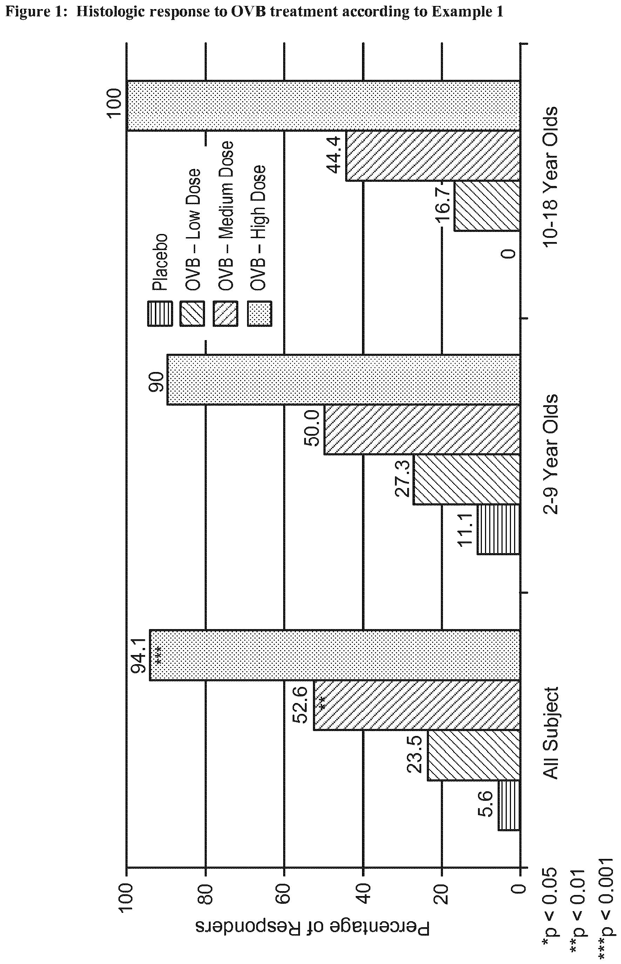

Histologic response to treatment is evaluated by measuring peak eosinophil levels in the esophageal mucosa. Histologic response is determined when the subject has maximum peak eosinophil counts of ≤ 6 per high power field in at all three sites (distal, mid, proximal) of the esophageal mucosa. Histologic response is observed by treatment group and age group is summarized in Figure 1.

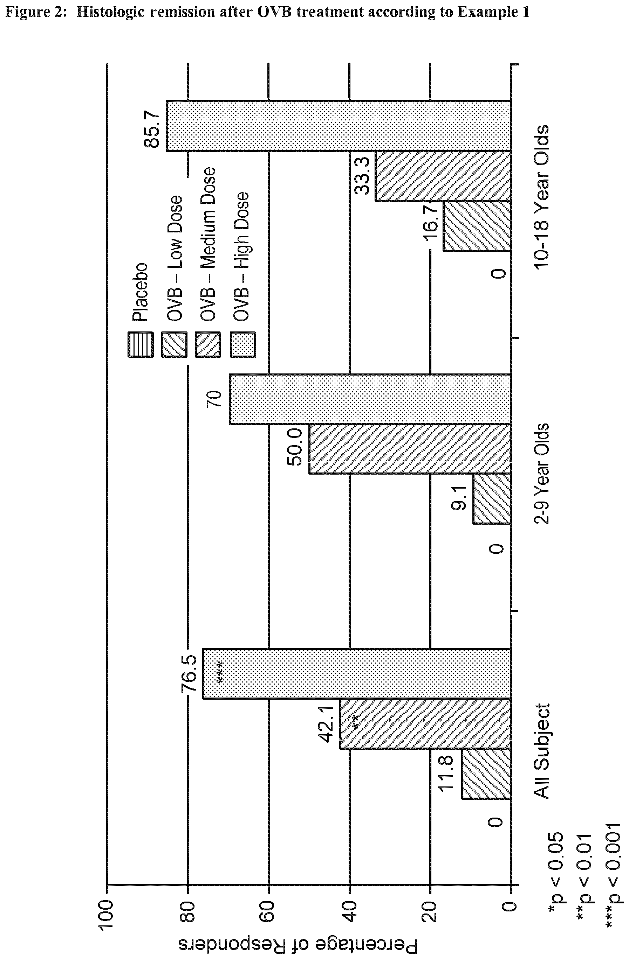

Histologic remission is determined when the subject has maximum peak eosinophil counts of ≤ 1 per high power field in at all three sites (distal, mid, proximal) of the esophageal mucosa. Histologic remission is observed by treatment group and age group is summarized in Figure 2.

Table 4A illustrates percent responders and percent remission in subjects:

**Table 4A.**

| Efficacy Parameter | Placebo N=18 | Low Dose N=17 | Medium Dose N=19 | High Dose N=17 |
|---|---|---|---|---|
| % Subjects with Histologic Response (Peak Eos ≤6/HPF) | 5.6 | 23.5 | 52.6 | 94.1 |
| - p-value (OBS vs Placebo) | - | 0.1786 | *0.0090* | *0.0001* |
| % Subjects with Histologic Remission (Peak Eos ≤1/HPF) | 0 | 11.8 | 42.1 | 76.5 |
| - p-value (OBS vs Placebo) | - | 0.4597 | *0.0042* | <*0.0001* |

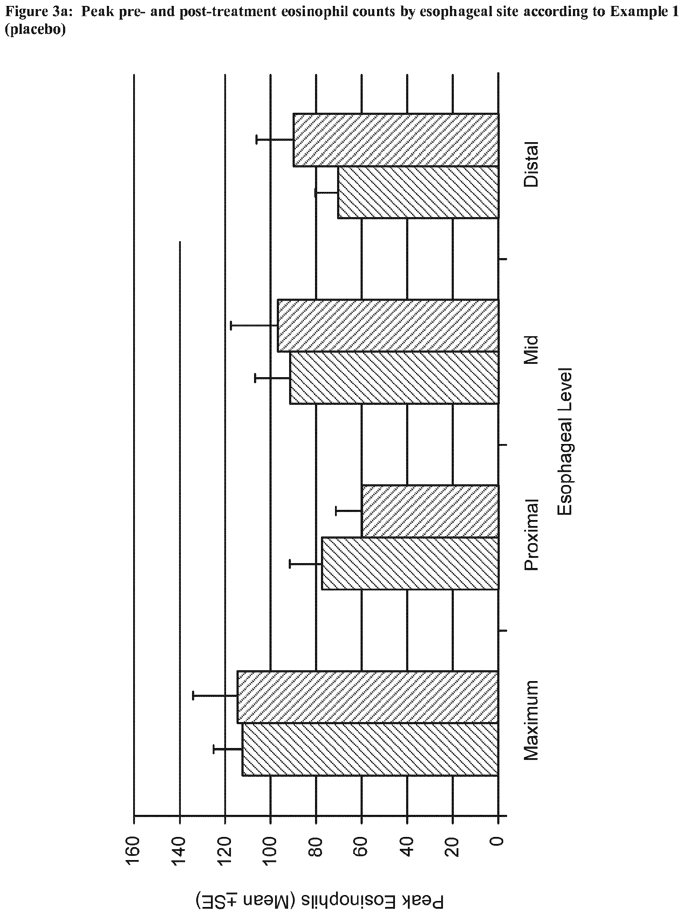

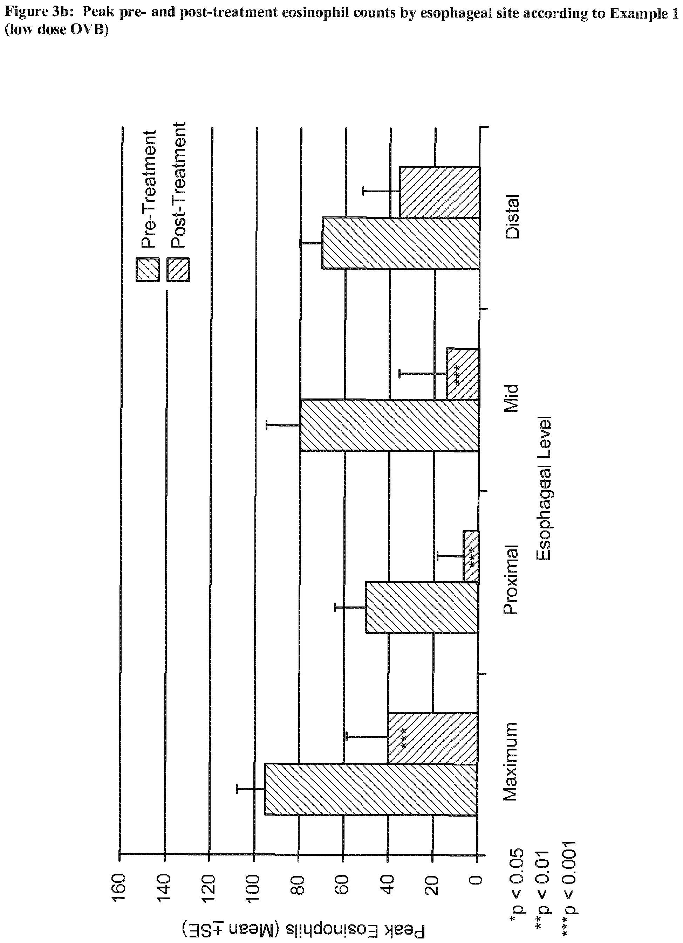

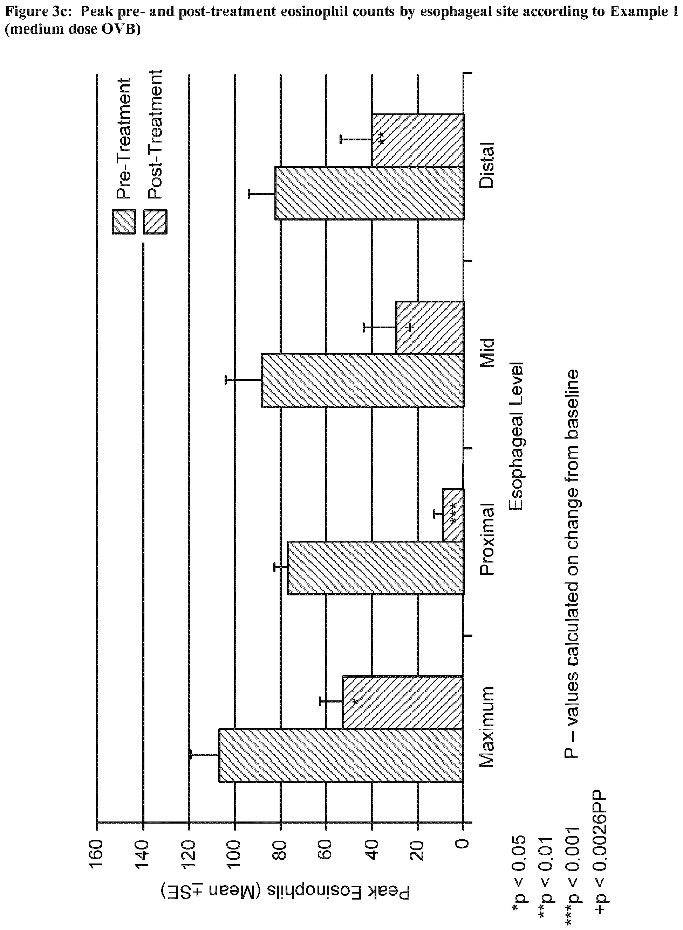

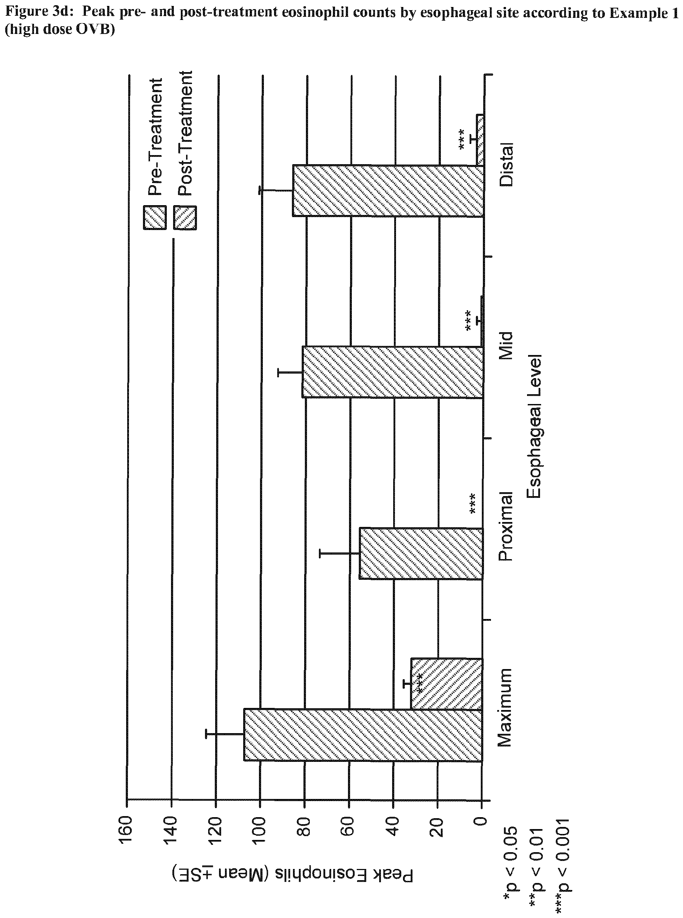

Peak pre- and post-treatment eosinophil counts by esophageal site are shown in Figures 3a-d.

Endoscopies are also taken, and each site of the esophagus (proximal, mid, distal) is evaluated based on the following four categories: 1) pallor and diminished vascular markings; 2) furrowing with thickened mucosa; 3) presence of white mucosal plaques; and 4) concentric rings or strictures. Each category is scored from 0 to 2, for a maximum score of 8. For each category, one point is allocated if 1 or 2 esophageal sites are involved, and two points for pan-esophageal involvement. Endoscopy scores for each treatment group are summarized in Table 4B below. Mean eosinophil counts for each treatment group are summarized in Table 4C below.

**Table 4B.**

| **Visit** | **Statistic** | **Placebo (n=18)** | **OVB Low Dose (n=17)** | **OVB Med Dose (n=19)** | **OVB High Dose (n=17)** |
|---|---|---|---|---|---|
| Baseline | Mean (SD) | 3.8 (1.8) | 2.9 (1.7) | 3.2 (1.5) | 3.4 (1.5) |
| Final Treatment Evaluation (Week 12) | Mean (SD) | 3.2 (1.9) | 2.0 (1.8) | 1.9 (1.6) | 1.2 (1.2) |
| Change from Baseline | Mean (SD) | -0.6 (2.3) | -0.9 (1.9) | -1.3 (2.2) | -2.2 (1.8) |
| | p-value | ---- | p=0.11 | p=0.026 | p=0.001 |

**Table 4C.**

| **Visit** | **Statistic** | **Placebo (n=18)** | **OVB Low Dose (n=17)** | **OVB Med Dose (n=19)** | **OVB High Dose (n=17)** |
|---|---|---|---|---|---|
| Baseline | Mean (SD) | 112.7 (52.03) | 95.6 (52.88) | 107.7 (30.74) | 108.4 (66.89) |
| Final Treatment Evaluation (Week 12) | Mean (SD) | 114.6 (81.92) | 39.5 (43.93) | 52.1 (72.05) | 3.5 (12.53) |
| Change from Baseline | Mean (SD) | 1.9 (78.09) | -56.2 | -55.7 (79.07) | -104.8 |
| | | | (48.14) | | (69.98) |
| Percent Change from Baseline | Mean (SD) | 7.93 (84.16) | -52.62 | -44.02 | -94.75 |
| | p-value vs. placebo | ---- | (51.22) | (89.11) | (19.62) |
| | | | 0.01 | 0.02 | <0.0001 |

### Symptom Results/Response:

Symptom response to treatment is evaluated by calculating a Symptom Score for each subject at weeks 0 (baseline), 2, 4, 8, and 12 (final) of treatment. The Symptom Score is calculated as follows:
(a) determining if the subject exhibits a symptom in the following categories: 1) heartburn; 2) abdominal pain; 3) nocturnal awakening with symptoms; 4) nausea, regurgitation or vomiting; 5) anorexia or early satiety; or 6) dysphagia, odynophagia, or food impaction;
(b) scoring each condition category on a 0 to 3 scale, wherein 0 = individual exhibits none of the symptoms in the condition category; 1 = symptoms limited to 1-3 days or no symptoms in the condition category because coping behaviors are used by the individual; 2 = individual exhibits symptoms for greater than 3 days, with or without minor coping behaviors; and 3 = symptoms in the condition category interfered with activities of daily living or symptoms persisted and required major coping behaviors; and
(c) calculating a total score by adding the score for each condition category.

Symptom Response in this study is defined by the subject's final Symptom Score is at least 50% lower than the subject's baseline Symptom Score. In some embodiments, a Symptom Response is at least 10% lower, at least 25% lower, at least 30% lower, at least 40% lower, at least 50% lower, at least 60% lower, at least 70% lower, at least 75% lower, at least 80% lower, at least 90% lower, at least 95% lower, or 100% lower than the subject's baseline Symptom Score. Determination of a Symptom Response can be determined in any suitable manner, e.g., by collecting data from the patient/subject (e.g., by use of a diary for symptom scoring), care provider, or investigator. A baseline and final Symptom Score can be determined in any suitable manner. For example, a baseline and/or final Symptom Score may include scoring averages or total scores measured the course of a few days, a week, multiple weeks, months, or the like (e.g., wherein the scores are obtained daily, weekly, etc.).

### Safety:

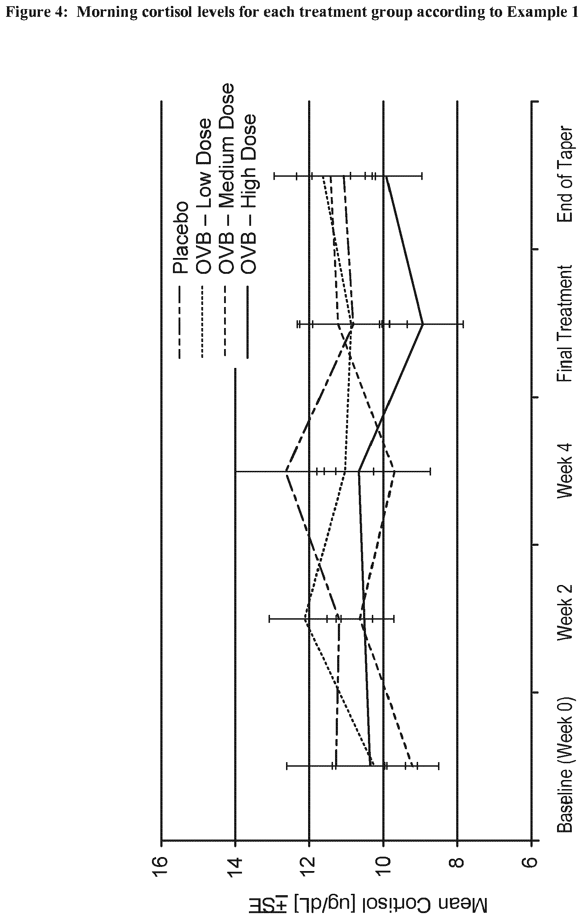

Morning cortisol levels for each treatment group are measured and are summarized in Figure 4. There are no statistically significant differences in morning cortisol values among the treatment groups at any timepoint. In addition, adverse events are evaluated and the number of subjects in relation to the type of adverse event are summarized in Table 5 below.

**Table 5.**

| **System Organ Class** | **Placebo (N=21) n (%)** | **OVB Low Dose (N=21) n (%)** | **OVB Med Dose (N=19) n (%)** | **OVB High Dose (N=20) n (%)** |
|---|---|---|---|---|
| Overall | 13 (61.9%) | 14 (66.7%) | 16 (84.2%) | 15 (75.0%) |
| Gastrointestinal | 3 (14.3%) | 3 (14.3%) | 5 (26.3%) | 5 (25.0%) |
| General Disorders | 3 (14.3%) | 2 (9.5%) | 5 (26.3%) | 5 (25.0%) |
| Immune System | 1 (4.8%) | 1 (4.8%) | 1 (5.3%) | 0 (0%) |
| Infections/Infestati ons | 6 (28.6%) | 9 (42.9%) | 10 (52.6%) | 5 (25.0%) |
| Injury/Procedural | 3 (14.3%) | 1 (4.8%) | 1 (5.3%) | 3 (15.0%) |
| Nervous System | 2 (9.5%) | 2 (9.5%) | 4 (21.1%) | 3 (15.0%) |
| Psychiatric | 2 (9.5%) | 2 (9.5%) | 2 (10.5%) | 0 (0%) |
| Respiratory -- Cough | 3 (14.3%) | 5 (23.8%) | 6 (31.6%) | 6 (30.0%) |
| Skin/Subcutaneous | 0 (0%) | 3 (14.3%) | 3 (15.8%) | 3 (15.0%) |

### Pharmacokinetics:

Blood samples are collected from each subject at pre-dose (0) and at 0.5, 1, 2, 3, 4, 6, and 8 hours post-dose. Pharmacokinetic parameters AUC, Cₘₐₓ, Tₘₐₓ, and T_{1/2} are measured from the blood samples. The lower limit of quantification (LLOQ) is 20 pg/mL when 0.2 mL samples are used. Results are summarized in Table 6 below.

**Table 6.**

| **Parameter** | **Statistic** | **OVB Low Dose** | **OVB Med Dose** | **OVB High Dose** |
|---|---|---|---|---|
| AUC₀₋ₗₐₛₜ (hr^{∗}pg/mL) | n | 9 | 15 | 13 |
| | Mean (SD) | 919.7 (611.9) | 3544.0 | 3308.5 |
| | Median | 943.0 | (2217.9) | (1623.3) |
| | | | 2670.0 | 2700.0 |
| Cₘₐₓ (pg/mL) | n | 9 | 15 | 13 |
| | Mean (SD) | 327.0 (303.4) | 1017.5 (636.8) | 960.8 (574.1) |
| | Median | 209.0 | 812.0 | 662.0 |
| Tₘₐₓ (hours) | n | 9 | 15 | 13 |
| | Mean (SD) | 0.97 (0.47) | 0.90 (0.39) | 1.15 (0.52) |
| | Median | 1.00 | 1.00 | 1.00 |
| T_{1/2} (hours) | n | 8 | 15 | 13 |
| | Mean (SD) | 3.34 (0.75) | 3.97 (2.56) | 2.95 (1.07) |
| | Median | 3.40 | 3.24 | 2.52 |

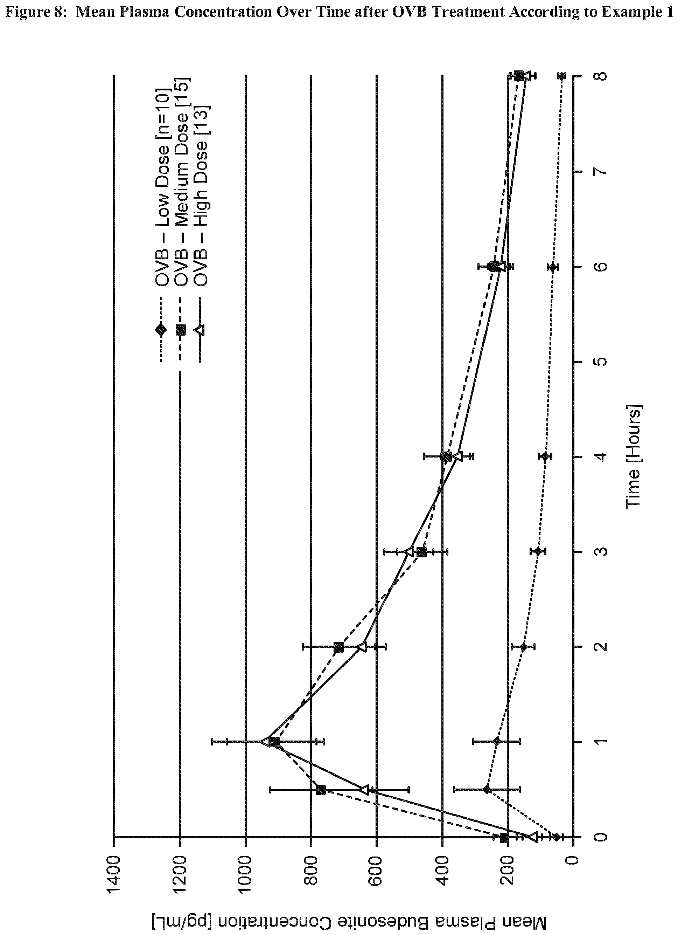

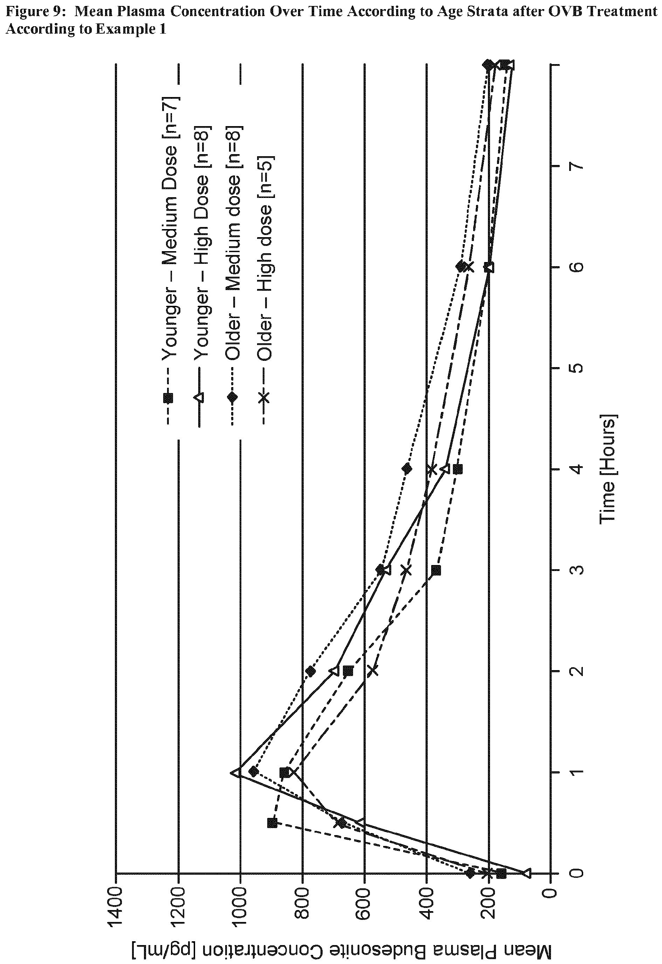

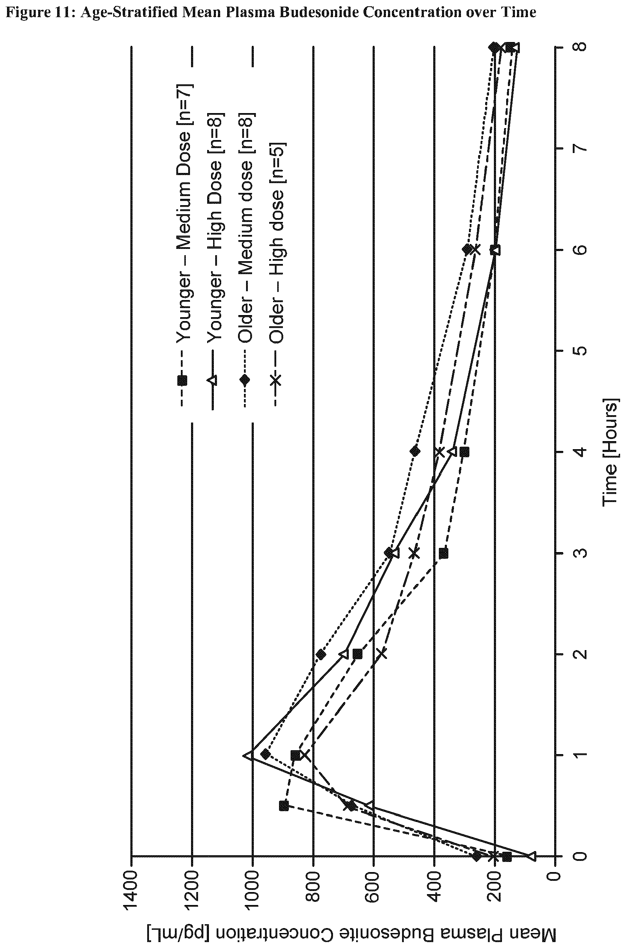

In addition, Figure 8 illustrates mean plasma concentration over time after OVB treatment, and Figure 9 illustrates mean plasma concentration over time according to age strata after OVB treatment. In Figure 9, "younger" is ages 2-9 and "older" is ages 10-18. Figure 11 illustrates age-stratified mean plasma budesonide concentration over time.

Pharmacokinetic parameters for a single dose in the medium and high dose groups are similar. Tₘₐₓ, T_{1/2}., and elimination rate constant are similar among low, medium, and high dose groups. Cₘₐₓ and AUC values increase in a dose proportional manner from the low dose to the medium and high dose groups. In the medium and high dose OVB groups, overall systemic drug exposure is comparable across the age groups.

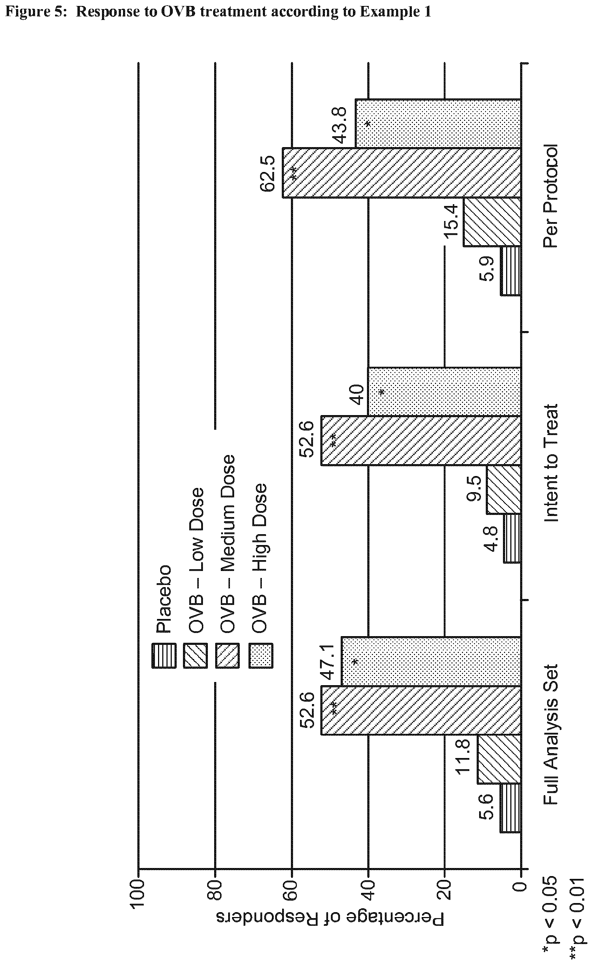

The percentage of responders is determined using the following methodology: when a subject has maximum peak eosinophil counts of ≤ 6 per high power field in at all three sites (distal, mid, proximal) of the esophageal mucosa, and the subject's final Symptom Score is at least 50% lower than the subject's baseline Symptom Score, a subject is considered a "responder." Primary Endpoint is determined using the number and/or percentage of responders. Primary Endpoint response to therapy described herein (including both a Histologic Response and Symptom Response) is illustrated in Figure 5 by treatment group. Figure 7 illustrates subjects achieving response (response for histology variable was defined as post-treatment peak eosinophil count ≤ 6/HPF; response for all other variables defined as a ≥ 50% reduction from baseline score) and remission (remission for histology variable was defined as post-treatment peak eosinophil count ≤ 1/HPF; remission for all other variables defined as a post-treatment score of 0) for selected study endpoints. Number of subjects that reached primary endpoint by treatment group and age are summarized in Table 7 below.

**Table 7.**

| **Treatment Group** | **Placebo** | **Low (0.05 mg/mL OVB qhs)** | **Medium (0.2 mg/mL OVB qhs)** | **High (0.2 mg/mL OVB bid)** |
|---|---|---|---|---|
| All Subjects Children 2-18 n/N(%) | 1/18 (5.6%) | 2/17 (11.8%) | 10/19 (52.6%) | 8/17 (47.1%) |
| Children Age 2-9, 7 mL n/N (%) | 1/9 (11.1%) | 2/11 (18.2%) | 6/10 (60%) | 3/10 (30%) |
| Children Age 10-18, 10 mL n/N(%) | 0/9 (0%) | 0/6 (0%) | 4/9 (44.4%) | 5/7 (71.4%) |

Subjects treated with medium dose OVB demonstrated a statistically significant response (53%) compared to placebo patients (6%) in the primary endpoint. Subjects in the medium and high dose treatment groups demonstrated statistically significant histological response and remission compared to placebo, where ^{∗} p < 0.05 and ^{∗∗} p < 0.01. Further, subjects in medium and high dose treatment groups demonstrated significant histological response and remission compared to low dose treatment groups.

### Alternative Methods for Measuring Response:

Response to treatment may also or alternatively be evaluated by calculating an Epithelial Score for each subject, e.g., at weeks 0 (baseline) and 12 (final) of treatment. The Epithelial Score is calculated as follows: (a) determining by visual inspection, *e*.*g*., by endoscopy, whether the individual's esophagus exhibits a condition in one of the following condition categories in the table below; (b) scoring each condition category according to the table below; and (c) determining a total epithelial score by adding the score for each condition category.

| Condition Category | Score | |
|---|---|---|
| Basal Layer Hyperplasia | None | 0 |
| | > 50% total epithelial thickness | 1 |
| | < 50% total epithelial thickness | 2 |
| Prevalence of Hyperplasia | N/A | 0 |
| | < 50% total biopsy volume | 1 |
| | > 50% total biopsy volume | 2 |
| Dilated Intercellular Spaces | Absent | 0 |
| | Present | 1 |
| Prevalence of Dilated Intercellular Spaces | N/A | 0 |
| | < 50% total biopsy volume | 1 |
| | > 50% total biopsy volume | 2 |
| Eos Microabscesses | None | 0 |
| | 1-2 microabscesses | 1 |
| | 3-4 microabscesses | 2 |
| | ≥ 5 microabscesses | 3 |
| Surface Layering | None | 0 |
| | < 50% total biopsy volume | 1 |
| | > 50% total biopsy volume | 2 |

The results are shown in Table 8 below.

**Table 8.**

| **Visit** | **Statistic** | **Placebo** | **OVB Low Dose** | **OVB Med Dose** | **OVB High Dose** |
|---|---|---|---|---|---|
| Baseline | n | 20 | 19 | 16 | 18 |
| | Mean | 4.83 (1.88) | 5.16 (2.07) | 5.09 (2.66) | 5.26 (2.23) |
| | (SD) | | | | |
| Final Treatment Evaluation (Week 12) | n | 17 | 17 | 19 | 17 |
| | Mean | 4.82 (2.91) | 1.45 (1.74) | 1.23 (1.69) | 0.20 (0.51) |
| | (SD) | | | | |
| Change from Baseline | n | 16 | 15 | 15 | 16 |
| | Mean | -0.23 (2.60) | -3.61 (2.78) | -3.74 (3.38) | -4.91 (2.37) |
| | (SD) | ---- | *p*<*0.0001* | *p*<*0.0001* | *p*<*0.0001* |
| | p-value | | | | |

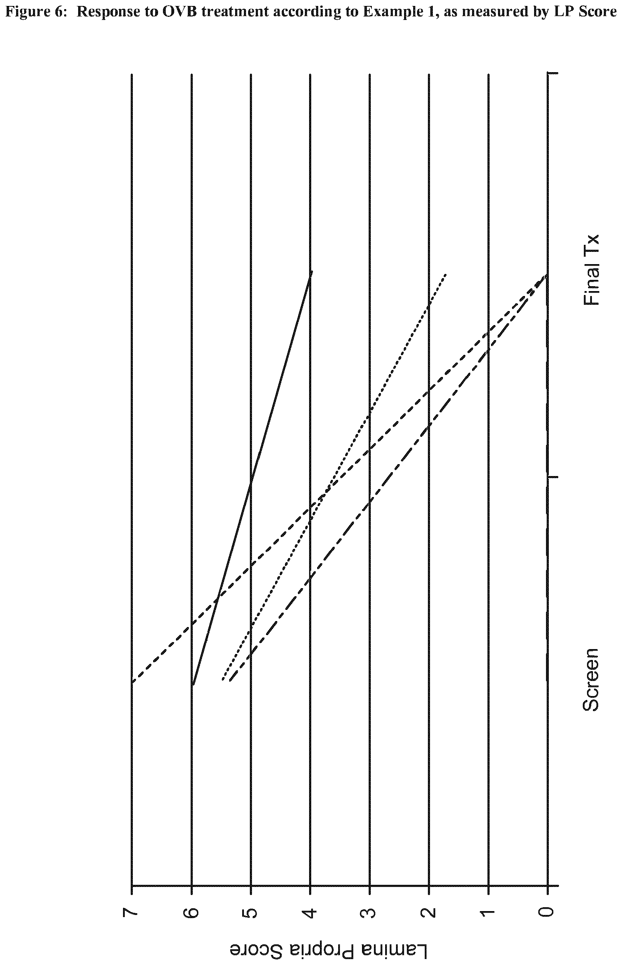

Response to treatment may also be evaluated by calculating a LP Score for each subject at weeks 0 (baseline) and 12 (final) of treatment. The LP Score is calculated as follows: (a) determining, for example by biopsy, whether the individual's esophagus exhibits a condition in one of the following condition categories in the table below; (b) scoring each condition category according to the table below; and (c) determining a total LP score by adding the score for each condition category. Figure 6 illustrates one embodiment of the invention comprising LP Score response to OVB treatment according to Example 1.

| Condition Category | Score | |
|---|---|---|
| Lamina Propria Fibrosis | None | 0 |
| | Mild | 1 |
| | Moderate | 2 |
| | Marked | 3 |
| Fibrosis Distribution | N/A | 0 |
| | Superficial | 1 |
| | Superficial and Deep | 2 |
| Prevalence of Fibrosis | N/A | 0 |
| | < 50% lamina propria volume | 1 |
| | > 50% lamina propria volume | 2 |

### Example 2: Endoscopy and Biopsy

As but one possible method, endoscopy is performed using an endoscope (by RD) and pan-esophageal, gastric and duodenal biopsies are taken. Two mucosal biopsies are taken from the proximal esophagus (3 cm below the crycopharyngeus muscle), distal esophagus (3 cm above the gastroesophageal junction (GEJ), and mid-esophagus (midpoint between the crycopharyngeus muscle and the GEJ).

Biopsies are processed routinely and evaluated by a pediatric pathologist (RN). The highest number of eosinophils per ×400 high power field are counted. Basal zone hyperplasia (BZH) is reported when basal zone cells extend towards the luminal surface of the epithelium (>25% of epithelial thickness).

### Example 3: Esophagogastroduodenoscopy and Biopsy

As but one possible method, Esophagogastroduodenoscopy (EGD) with esophageal, gastric and duodenal biopsies is required for study participation. It may be performed by a referring physician prior to the Screening Visit or it may be performed during the Screening Period, or is performed in the 6 weeks prior to the Baseline Visit. A repeat endoscopy with esophageal biopsies is performed at the Final Treatment Evaluation, or earlier if the subject is prematurely withdrawn from the study.

Endoscopic findings in the esophagus are recorded with respect to four categories: 1) pallor and diminished vascular markings; 2) furrowing with thickened mucosa; 3) presence of white mucosal plaques; and 4) concentric rings or strictures.

An endoscopy score for each category are calculated; zero points if no esophageal sites are involved, one point allocated if 1 or 2 esophageal sites are involved, and two points for pan-esophageal involvement, with a maximum endoscopy score of 8.

Esophageal biopsies are performed within 6 weeks prior to the Baseline Visit by an endoscopist. If the biopsy is performed by a referring physician, the pathology report and/or slides are reviewed at the study site in order to assess study eligibility. Multiple specimens (at least 2 biopsies from each of 3 levels) are obtained from the proximal (3 cm below the crycopharyngeus muscle), distal (3 cm above the gastroesophageal junction) and mid esophagus (mid-point between the crycopharyngeus muscle and the gastroesophageal junction). In addition, biopsies are taken from the stomach and duodenum as follows: gastric body (greater curvature): 2, gastric antrum: 2, duodenum (third part or distal): 2. If the pre-treatment biopsy identifies disease in the stomach and/or duodenum, the subject will be excluded from the study, but does not mean subjects cannot be treated according to the methods of the invention.

### Example 4: Symptom Score

### Administration Guidelines

Clinical symptoms of study subjects with EoE will be elicited by a physician investigator, patient/subject, or caregiver using a semi-structured instrument that includes one or more of the following six symptom domains: 1) heartburn; 2) abdominal pain; 3) nocturnal awakening with symptoms; 4) nausea, regurgitation or vomiting; 5) anorexia or early satiety; or 6) dysphagia, odynophagia, or food impaction. For example, symptoms will be reported and/or recorded by the investigator, patient/subject (e.g., by use of an electronic journal), or caregiver, with respect to frequency of symptoms, disruptiveness of symptoms, and disruptiveness of coping behaviors.

Based on severity of the symptom, a score may be individually assigned to the symptom, up to a maximum (e.g., a score range of 0-3). For example, if all six symptoms are scored and the maximum score for each symptom domain will be 3; the maximum total score will be 18.

Symptoms: Symptoms that interfere with usual daily activities will be considered disruptive. Symptoms may be considered disruptive by the subject and primary caregiver based on frequency of symptoms (*e*.*g*., regurgitation at most meals [as opposed to several times in the past two weeks], or frequent interruption of sleep). Duration of symptoms may also contribute to disruptiveness. For example, food sticking in the esophagus for a few seconds, or a minute, may not be disruptive, but food sticking for 10 to 20 minutes may interfere with usual daily activities. Symptoms may be considered disruptive even if they are infrequent (*e*.*g*., vomiting in the school cafeteria; symptoms resulting in missed school days; and symptoms leading to an unscheduled visit to a doctor or emergency room).

### Symptom Definitions:

Dysphagia: Symptoms refer to difficulty with swallowing food or liquid, the sensation of food or liquid passing slowly, or food hanging up, sticking in, or obstructing the esophagus. Dysphagia refers to esophageal dysphagia rather than oropharyngeal dysphagia.

Odynophagia: Pain in the esophagus associated with swallowing of food or liquid; in this clinical study, this symptom refers to esophageal odynophagia rather than oropharyngeal odynophagia (*e*.*g*., as may occur with acute pharyngitis). The odynophagia may occur with or without dysphagia.

Food impaction: A condition in which food becomes stuck in the esophagus and requires some intervention by the patient or by medical personnel to dislodge the food. Patient intervention may include such actions as induced gagging, coughing, or retching.

Heartburn: Burning or painful sensation in the chest (especially the mid-chest area behind the sternum); child may complain that the chest "hurts."

Abdominal pain: Pain or discomfort in the abdomen (i.e., below the diaphragm) with any of several qualities (*e*.*g*., crampy, persistent, achy, dull, sharp) or in any location (*e*.*g*., epigastric, diffuse, periumbilical). In very young children, abdominal pain or discomfort may manifest itself as unexplained irritability or persistent crying such as occurs with colic.

Nausea: A sensation of discomfort in the stomach associated with an urge to vomit; this symptom may be described as an "upset stomach."

Regurgitation: The process of food or liquid coming up into the throat or mouth (but not being vomited or expelled from the mouth). There may be a burning sensation in the throat. The food or liquid may be tasted or there may be an acid taste in the mouth.

Vomiting: The process of expelling food or liquid from the mouth; this may or may not be forceful (*e*.*g*., projectile vomiting vs. spitting up in very young children).

Anorexia: Diminished or no appetite; lack of desire to eat.

Early satiety: Sensation of satiety or fullness after ingestion of only a small amount of food or a small portion of a meal.

Usual Daily Activities include, but are not limited to, school attendance and performance, after school activities, playing with friends, eating in public places, sleep, etc.

Coping Behaviors may be behaviors used to avoid or lessen symptoms or avoid embarrassment associated with symptoms. Such behaviors may include: taking medication (other than study medication), taking antacids, cutting food into small pieces, eating less than normal amounts of food, vomiting, taking a drink, chewing food longer than normal, avoiding certain foods, etc. Coping behaviors that interfere with usual daily activities will be considered disruptive. Coping behaviors that are solely habitual and no longer address ongoing symptoms should be excluded from consideration when answering questions about coping behaviors.

While certain embodiments have been shown and described herein, it will be apparent to those skilled in the art that such embodiments are provided by way of example only. Numerous variations, changes, and substitutions will now occur to those skilled in the art and are considered to be within the scope of the disclosure herein. It should be understood that various alternatives to the embodiments of the invention described herein may be employed in practicing the invention. It is intended that the following claims define the scope of the invention and that methods and structures within the scope of these claims and their equivalents be covered thereby.

Also described herein are the following numbered clauses:
1. An orally administered composition comprising an effective amount of a corticosteroid for use in reducing eosinophilic infiltration of the esophagus in an individual in need thereof, or for inducing remission in an individual in need thereof.
2. The composition for use of clause 1, wherein, when eosinophilic infiltration of the esophagus is reduced, the individual has 1 or fewer eosinophils per high power field in the esophageal mucosa.
3. The composition for use of clause 1, wherein, when eosinophilic infiltration of the esophagus is reduced, the individual has 1 or fewer eosinophils per high power field in the distal esophageal mucosa, the mid esophageal mucosa, the proximal esophageal mucosa, or a combination thereof.
4. The composition for use of clause 3, wherein when eosinophilic infiltration of the esophagus is reduced, the individual has 1 or fewer eosinophils per high power field in the distal esophageal mucosa, the mid esophageal mucosa, and the proximal esophageal mucosa.
5. The composition for use of clause 1, wherein, when eosinophilic infiltration of the esophagus is reduced, an endoscopy score is determined by:
   (a) visually inspecting one or more sites of the esophagus for the following conditions 1) pallor and diminished vascular markings; 2) furrowing with thickened mucosa; 3) presence of white mucosal plaques; and 4) concentric rings or strictures;
   (b) scoring each condition,
      and has an endoscopy score reduced by at least 50% compared to pretreatment score.
6. The composition for use of clause 1, wherein, when eosinophilic infiltration of the esophagus is reduced, a symptom score is determined by:
   (a) determining if the individual exhibits a symptom in any one or more of the following conditions category or categories: 1) heartburn; 2) abdominal pain; 3) nocturnal awakening with symptoms; 4) nausea, regurgitation or vomiting; 5) anorexia or early satiety; or 6) dysphagia, odynophagia, or food impaction;
   (b) scoring each condition category,
      and has a symptom score reduced by at least 50% compared to pretreatment score.
8. The composition for use of clause 1, wherein the individual is suffering from esophageal inflammation or symptoms thereof.
9. The composition for use of clause 1, wherein the individual is suffering from eosinophilic esophagitis.
10. The composition for use of clause 1, wherein the corticosteroid is a topically active corticosteroid.
11. The composition for use of clause 1, wherein the corticosteroid is aclometasone, amcinomide, beclometasone, betamethasone, budesonide, ciclesonide, clobetasol, clobetasone, clocortolone, cloprednol, cortivazol, deflazacort, deoxycorticosterone, desonide desoximetasone, dexamethasone, diflorasone, diflucortolone, difluprednate, fluclorolone, fludrocortisone, fludroxycortide, flumetasone, flunisolide, fluocinolone acetonide, fluocinonide, fluocortin, fluocortolone, fluorometholone, fluperolone, fluticasone, fuprednidene, formocortal, halcinonide, halometasone, hydrocortisone aceponate, hydrocortisone buteprate, hydrocortisone butyrate, loteprednol, medrysone, meprednisone, methylprednisolone, methylprednisolone aceponate, mometasone, paramethasone, prednicarbate, prednisone, prednisolone, prednylidene, remexolone, tixocortol, triamcinolone, ulobetasol, or a pharmaceutically acceptable salt or ester thereof, or a combination thereof.
12. The composition for use of clause 1, wherein the corticosteroid is budesonide, fluticasone, mometasone, desonide, ciclesonide, triamcinolone, beclomethasone, or a pharmaceutically acceptable ester thereof, or a combination thereof.
13. The composition for use of clause 1, wherein the corticosteroid is budesonide.
14. The composition for use of clause 1, wherein the individual is 12-17, 12-50, or 10 or more years old and the corticosteroid is administered in a composition having a total volume of about 8 to about 12 mL, or about 10 mL.
15. The composition for use of clause 12, wherein the individual is less than 10 years old and the corticosteroid is administered in a composition having a total volume of about 5 to about 10 mL, or about 7 mL.
16. The composition for use of clause 1, wherein the corticosteroid is administered to the individual in an amount of about 0.035 mg or more per day.
17. The composition for use of clause 1, wherein the corticosteroid is administered to the individual in an amount of about 0.035 mg to about 10 mg per day.
18. The composition for use of clause 1, wherein the corticosteroid is administered to the individual in an amount of about 2 mg to about 4 mg per day.
19. The composition for use of clause 1, wherein the corticosteroid is administered to the individual in an amount of about 0.35 mg, 0.5 mg, 1.4 mg, 2 mg, or 4 mg per day.
20. The composition for use of clause 1, wherein the corticosteroid is administered to the individual at least once a day.
21. The composition for use of clause 1, wherein the corticosteroid is administered to the individual once a day.
22. The composition for use of clause 1, wherein the corticosteroid is administered to the individual twice a day.
23. The composition for use of clause 1, wherein the corticosteroid is administered to the individual in the form of a liquid composition.
24. The composition for use of clause 23, wherein the composition comprises corticosteroid in an amount greater than 0.035 mg per mL, greater than 0.05 mg per mL or greater than 0.07 mg per mL of total volume of the composition.
25. The composition for use of clause 24, wherein the composition comprises about 0.1 mg to about 0.2 mg corticosteroid per milliliter of total volume of the composition.
26. The composition for use of clause 24, wherein the composition comprises about 0.2 mg corticosteroid per milliliter of total volume of the composition.
27. The composition for use of clause 24, wherein about 5 to about 15 milliliters of the composition is administered to the individual per dose.
28. The composition for use of clause 24, wherein about 7 to about 10 milliliters of the composition is administered to the individual per dose.
29. The composition for use of clause 1, wherein the corticosteroid is administered to the individual in the form of a pharmaceutical composition comprising an effective amount of the corticosteroid and at least one pharmaceutically acceptable excipient.
30. The composition for use of clause 29, wherein the pharmaceutical composition is in the form of a solution, suspension, emulsion, syrup, slurry, dispersion, elixir, tonic, orally dissolving tablet, lozenge, powder, granules, micropellets, nanopellets, microparticles, nanoparticles, tablet, effervescent tablet, melting tablet, disintegrating tablet, orally disintegrating tablet, foam, gel, solid solution, solid formulation, solid disintegrating formulation, emulsion, liquid or semi-liquid solution, gum, wafer (*e*.*g*., dissolving or disintegrating), or a combination thereof, or a film or patch or the like.
31. The composition for use of clause 29, wherein the pharmaceutical composition comprises: a therapeutically effective amount of corticosteroid, a preservative, a buffer, a surface active agent or a surfactant, an optional preservative, an optional flavoring agent, an optional sweetener, at least one additional excipient, and a liquid vehicle, or a therapeutically effective amount of the corticosteroid, edetate, citrate, polysorbate 80, an optional preservative, an optional flavoring agent, an optional sweetener, at least one additional excipient, and a liquid vehicle.
32. A composition comprising a corticosteroid for use in treating esophageal inflammation in an individual in need thereof, comprising:
   (a) orally administering to the individual according to a first daily dosing regimen comprising an amount of corticosteroid in an amount effective to reduce eosinophilic infiltration of the esophagus; and subsequently
   (b) orally administering to the individual according to a second daily dosing regimen comprising an amount of corticosteroid in an amount effective to maintain a reduced level of eosinophilic infiltration of the esophagus or symptoms associated therewith.
33. A composition comprising a corticosteroid for use in treating esophageal inflammation in an individual in need thereof, comprising:
   (a) orally administering to the individual according to a first daily dosing regimen comprising an amount of corticosteroid in an amount effective to reduce the individual's eosinophil count to 6 or fewer or 1 or fewer eosinophils per high power field in the esophageal mucosa; and
   (b) orally administering to the individual according to a second daily dosing regimen comprising an amount of corticosteroid in an amount effective to maintain the individual's eosinophil count at 6 or fewer or 1 or fewer eosinophils per high power field in the esophageal mucosa.
34. The composition for use of clause 32, wherein in step (a), the eosinophilic infiltration of the esophagus is reduced when the individual has 15 or fewer eosinophils per high power field in the esophageal mucosa.
35. The composition for use of clause 32, wherein in step (a), the eosinophilic infiltration of the esophagus is reduced when the individual has 15 or fewer, or 6 or fewer eosinophils per high power field in the esophageal mucosa.
36. The composition for use of clause 32, wherein in step (a), the eosinophilic infiltration of the esophagus is reduced when the individual has 6 or fewer, or 1 or fewer eosinophils per high power field in the esophageal mucosa.
37. The composition for use of clause 32, wherein, when eosinophilic infiltration of the esophagus is reduced, an endoscopy score is determined by:
   (a) visually inspecting one or more sites of the esophagus for one or more of the following conditions 1) pallor and diminished vascular markings; 2) furrowing with thickened mucosa; 3) presence of white mucosal plaques; and 4) concentric rings or strictures;
   (b) scoring each condition
      and the individual has an endoscopy reduced by at least 50% compared to pretreatment score.
38. The composition for use of clause 32, wherein the individual is suffering from eosinophilic esophagitis.
39. The composition for use of clause 32, wherein the corticosteroid is aclometasone, amcinomide, beclometasone, betamethasone, budesonide, ciclesonide, clobetasol, clobetasone, clocortolone, cloprednol, cortivazol, deflazacort, deoxycorticosterone, desonide desoximetasone, dexamethasone, diflorasone, diflucortolone, difluprednate, fluclorolone, fludrocortisone, fludroxycortide, flumetasone, flunisolide, fluocinolone acetonide, fluocinonide, fluocortin, fluocortolone, fluorometholone, fluperolone, fluticasone, fuprednidene, formocortal, halcinonide, halometasone, hydrocortisone aceponate, hydrocortisone buteprate, hydrocortisone butyrate, loteprednol, medrysone, meprednisone, methylprednisolone, methylprednisolone aceponate, mometasone, paramethasone, prednicarbate, prednisone, prednisolone, prednylidene, remexolone, tixocortol, triamcinolone, ulobetasol, or a pharmaceutically acceptable salt or ester thereof, or a combination thereof.
40. The composition for use of clause 39, wherein the corticosteroid is budesonide, fluticasone, mometasone, desonide, ciclesonide, triamcinolone, beclomethasone, or a pharmaceutically acceptable ester thereof, or a combination thereof.
41. The composition for use of clause 40, wherein the corticosteroid is budesonide.
42. The composition for use of clause 41, wherein (1) the first daily amount is 2-6 mg per day, and (2) the second daily amount is 1-2 mg per day or the daily maintenance dose is 0.25-2 mg per day.
43. The composition for use of clause 41, wherein the first daily dosing regimen comprises an amount administered in a 1-2 mg dose twice per day and the second daily dosing regimen comprises an amount administered in a 1-2 mg dose once per day.
44. The composition for use of clause 32, wherein step (a) or (b) is repeated for at least six months or 12 months.
45. The composition for use of clause 32, wherein step (a) or (b) is repeated for at least one month.
46. The composition for use of clause 32, wherein the individual is 10 or more years old and the corticosteroid is administered in a composition having a total volume of about 8 to about 12 mL, or about 10 mL.
47. The composition for use of clause 32, wherein the individual is less than 10 years old and the corticosteroid is administered in a composition having a total volume of about 5 to about 12 mL, or about 10 mL.
48. The composition for use of clause 32, wherein the corticosteroid in step (a) is administered to the individual twice a day.
49. The composition for use of clause 32, wherein the corticosteroid in step (b) is administered to the individual once a day.
50. The composition for use of clause 32, wherein the corticosteroid is administered to the individual in the form of a liquid composition.
51. The composition for use of clause 50, wherein the composition comprises 0.035 mg or more, 0.5 mg or more, about 0.07 mg or more, or about 0.1 mg or more corticosteroid per milliliter.
52. The composition for use of clause 50, wherein the composition comprises about 0.1 mg to about 0.2 mg corticosteroid per milliliter, or about 0.2 mg corticosteroid per milliliter.
53. The composition for use of clause 32, wherein:
   step (a) is repeated for about 3 months, and step (b) is repeated for about 3 months, or
   step (a) is repeated for about 2 months, and step (b) is repeated for about 3 months, or
   step (a) is repeated for about 3 months, and step (b) is repeated for about 2 months, or
   step (a) is repeated for about 1 month, and step (b) is repeated for about 2 months, or
   step (a) is repeated for about 1 month, and step (b) is repeated for about 1 month, or
   step (a) is repeated for about 4 weeks, and step (b) is repeated for about 4 weeks, or
   step (a) is repeated for about 2 weeks, and step (b) is repeated for about 2 weeks, or
   step (a) is repeated for about 3 months, and step (b) is repeated indefinitely, or
   step (a) is repeated for about 2 months, and step (b) is repeated indefinitely, or
   step (a) is repeated for about 1 month, and step (b) is repeated indefinitely, or
   step (a) is repeated for about 4 weeks, and step (b) is repeated indefinitely, or
   step (a) is repeated for about 2 weeks, and step (b) is repeated indefinitely.
54. The composition for use of clause 50, wherein about 5 to about 15 milliliters or about 7 to about 10 milliliters of the composition is administered to the individual per dose.
55. The composition for use of clause 32, wherein the corticosteroid is administered to the individual in the form of a pharmaceutical composition comprising an effective amount of the corticosteroid and at least one pharmaceutically acceptable excipient.
56. The composition for use of clause 55, wherein the pharmaceutical composition is in the form of a solution, suspension, emulsion, syrup, slurry, dispersion, elixir, tonic, orally dissolving tablet, lozenge, powder, granules, micropellets, nanopellets, microparticles, nanoparticles, tablet, effervescent tablet, melting tablet, disintegrating tablet, orally disintegrating tablet, foam, gel, solid solution, solid formulation, solid disintegrating formulation, emulsion, liquid or semi-liquid solution, gum, wafer (*e*.*g*., dissolving or disintegrating), or a combination thereof, or a film or patch or the like.
57. The composition for use of clause 55, wherein the pharmaceutical composition comprises: a therapeutically effective amount of corticosteroid, a preservative, a buffer, a surface active agent or a surfactant, an optional preservative, an optional flavoring agent, an optional sweetener, at least one additional excipient, and a liquid vehicle, or a therapeutically effective amount of the corticosteroid, edetate, citrate, polysorbate 80, an optional preservative, an optional flavoring agent, an optional sweetener, at least one additional excipient, and a liquid vehicle.
58. A composition comprising a corticosteroid for use in treating esophageal inflammation in an individual in need thereof, comprising:
   (a) orally administering to the individual according to a first daily dosing regimen comprising an amount of corticosteroid in an amount effective to reduce eosinophilic infiltration of the esophagus;
   (b) orally administering to the individual according to a second daily dosing regimen comprising an amount of corticosteroid in an amount effective to further reduce eosinophilic infiltration of the esophagus; and subsequently
   (c) orally administering to the individual according to a third daily dosing regimen comprising an amount of corticosteroid in an amount effective to maintain a reduced level of eosinophilic infiltration of the esophagus.
59. A composition comprising a corticosteroid for use in treating esophageal inflammation in an individual in need thereof, comprising:
   (a) orally administering to the individual according to a first daily dosing regimen comprising an amount of corticosteroid in an amount effective to reduce the individual's eosinophil count to 23 or fewer or 15 or fewer eosinophils per high power field in the esophageal mucosa; and
   (b) orally administering to the individual according to a second daily dosing regimen comprising an amount of corticosteroid in an amount effective to reduce the individual's eosinophil count to 15 or fewer, 6 or fewer or 1 or fewer eosinophils per high power field in the esophageal mucosa; and subsequently
   (c) orally administering to the individual according to a third daily dosing regimen comprising an amount of corticosteroid in an amount effective to maintain the individual's eosinophil count at 15 or fewer, 6 or fewer or 1 or fewer eosinophils per high power field in the esophageal mucosa.
60. The composition for use of clause 58, wherein in step (a), the eosinophilic infiltration of the esophagus is reduced when the individual has 23 or fewer, or 15 or fewer eosinophils per high power field in the esophageal mucosa.
61. The composition for use of clause 58, wherein in step (b), the eosinophilic infiltration of the esophagus is further reduced when the individual has 15 or fewer, or 6 or fewer, or 1 or fewer eosinophils per high power field in the esophageal mucosa.
62. The composition for use of clause 58, wherein, when eosinophilic infiltration of the esophagus is reduced, wherein an endoscopy score is determined by:
   (a) visually inspecting one or more sites of the esophagus for one or more of the following conditions 1) pallor and iminished vascular markings; 2) furrowing with thickened mucosa; 3) presence of white mucosal plaques; and 4) concentric rings or strictures;
   (b) scoring each condition,
      and the individual has an endoscopy score reduced by at least 50% compared to pretreatment score.
63. The composition for use of clause 58, wherein, when eosinophilic infiltration of the esophagus is reduced, a symptom score is determined by:
   (a) determining if the individual exhibits a symptom in any one or more of the following conditions category or categories: 1) heartburn; 2) abdominal pain; 3) nocturnal awakening with symptoms; 4) nausea, regurgitation or vomiting; 5) anorexia or early satiety; or 6) dysphagia, odynophagia, or food impaction;
   (b) scoring each condition category, and has a symptom score reduced by at least 50% compared to pretreatment score.
64. The composition for use of clause 58, wherein, when eosinophilic infiltration of the esophagus is reduced, a LP score is determined by:
   (a) determining whether the individual's esophagus exhibits lamina propria (LP) fibrosis; and
   (b) scoring the condition;
   and has a LP score reduced by at least 50% compared to pretreatment score.
65. The composition for use of clause 58, wherein the individual is suffering from eosinophilic esophagitis.
66. The composition for use of clause 58, wherein the corticosteroid is aclometasone, amcinomide, beclometasone, betamethasone, budesonide, ciclesonide, clobetasol, clobetasone, clocortolone, cloprednol, cortivazol, deflazacort, deoxycorticosterone, desonide desoximetasone, dexamethasone, diflorasone, diflucortolone, difluprednate, fluclorolone, fludrocortisone, fludroxycortide, flumetasone, flunisolide, fluocinolone acetonide, fluocinonide, fluocortin, fluocortolone, fluorometholone, fluperolone, fluticasone, fuprednidene, formocortal, halcinonide, halometasone, hydrocortisone aceponate, hydrocortisone buteprate, hydrocortisone butyrate, loteprednol, medrysone, meprednisone, methylprednisolone, methylprednisolone aceponate, mometasone, paramethasone, prednicarbate, prednisone, prednisolone, prednylidene, remexolone, tixocortol, triamcinolone, ulobetasol, or a pharmaceutically acceptable salt or ester thereof, or a combination thereof.
67. The composition for use of clause 66, wherein the corticosteroid is budesonide, fluticasone, mometasone, desonide, ciclesonide, triamcinolone, beclomethasone, or a pharmaceutically acceptable ester thereof, or a combination thereof.
68. The composition for use of clause 7, wherein the corticosteroid is budesonide.
69. The composition for use of clause 68, wherein the first daily dosing regimen comprises an amount of 1-2 mg per day, the second daily dosing regimen comprises an amount of 2-6 mg per day, and the third daily dosing regimen comprises an amount of 0.035-2 mg per day.
70. The composition for use of clause 68, wherein the first daily dosing regimen comprises an amount administered in a 1-2 mg dose once per day, the second daily dosing regimen comprises an amount administered in a 2-3 mg dose twice per day, and the third daily dosing regimen comprises an amount administered in a 1-2 mg dose once per day.
71. The composition for use of clause 58, wherein:
   step (a) is repeated for about 3 months, and step (b) is repeated for about 3 months, or step (a) is repeated for about 2 months, and step (b) is repeated for about 3 months, or step (a) is repeated for about 3 months, and step (b) is repeated for about 2 months, or step (a) is repeated for about 1 month, and step (b) is repeated for about 2 months, or
   step (a) is repeated for about 1 month, and step (b) is repeated for about 1 month, or
   step (a) is repeated for about 4 weeks, and step (b) is repeated for about 4 weeks, or
   step (a) is repeated for about 2 weeks, and step (b) is repeated for about 2 weeks
72. The composition for use of clause 58, wherein step (a), (b), or (c) is repeated for at least one month, or at least six months.
73. The composition for use of clause 58, wherein the individual is 10 or more years old and the corticosteroid is administered in a composition having a total volume of about 8 to about 12 mL, or about 10 mL.
74. The composition for use of clause 58, wherein the individual is less than 10 years old and the corticosteroid is administered in a composition having a total volume of about 5 to about 12 mL, or about 7 mL.
75. The composition for use of clause 58, wherein the corticosteroid in step (b) is administered to the individual twice a day.
76. The composition for use of clause 58, wherein the corticosteroid in step (a) or (c) is administered to the individual once a day.
77. The composition for use of clause 58, wherein the corticosteroid is administered to the individual in the form of a liquid composition.
78. The composition for use of clause 77, wherein the composition comprises 0.035 mg or more, 0.5 mg or more, about 0.07 mg or more, or about 0.1 mg or more corticosteroid per milliliter.
79. The composition for use of clause 77, wherein the composition comprises about 0.1 mg to about 0.2 mg corticosteroid per milliliter.
80. The composition for use of clause 77, wherein the composition comprises about 0.2 mg corticosteroid per milliliter.
81. The composition for use of clause 77, wherein about 5 to about 15 milliliters or about 7 to about 10 milliliters of the composition is administered to the individual per dose.
82. The composition for use of clause 58, wherein the corticosteroid is administered to the individual in the form of a pharmaceutical composition comprising an effective amount of the corticosteroid and at least one pharmaceutically acceptable excipient.
83. The composition for use of clause 82, wherein the pharmaceutical composition is in the form of a solution, suspension, emulsion, syrup, slurry, dispersion, elixir, tonic, orally dissolving tablet, lozenge, powder, granules, micropellets, nanopellets, microparticles, nanoparticles, tablet, effervescent tablet, melting tablet, disintegrating tablet, orally disintegrating tablet, foam, gel, solid solution, solid formulation, solid disintegrating formulation, emulsion, liquid or semi-liquid solution, gum, wafer (e.g., dissolving or disintegrating), or a combination thereof, or a film or patch or the like.
84. The composition for use of clause 82, wherein the pharmaceutical composition comprises: a therapeutically effective amount of corticosteroid, a preservative, a buffer, a surface active agent or a surfactant, an optional preservative, an optional flavoring agent, an optional sweetener, at least one additional excipient, and a liquid vehicle, or a therapeutically effective amount of the corticosteroid, edetate, citrate, polysorbate 80, an optional preservative, an optional flavoring agent, an optional sweetener, at least one additional excipient, and a liquid vehicle.
85. The composition for use of clause 1, wherein the individual's morning cortisol levels during administration of the corticosteroid are in the range of 5-20 µg/dL or 8-14 µg/dL or does not fall below 5 µg/dL.
86. The composition for use of clause 1, wherein the reduction of eosinophilic infiltration of the esophagus is proportional to the amount of corticosteroid administered to the individual, or where greater reduction of eosinophilic infiltration of the esophagus occurs when a greater amount of corticosteroid is administered to the individual.
87. An orally administered composition comprising an effective amount of a corticosteroid for use in reducing eosinophilic infiltration of the esophagus in an individual in need thereof, or for inducing remission in an individual in need thereof, wherein plasma:
   AUC₀₋₈ₕ is less than 4000 h^{∗}pg/mL, about 2000 to about 4000 h^{∗}pg/mL, about 2500 to about 3000 h^{∗}pg/mL, or about 2700 h^{∗}pg/mL; and/or
   Cₘₐₓ is less than 1500 pg/mL, about 500 to about 1500 pg/mL, about 600 to about 900 pg/mL, or about 700 pg/mL; and/or
   Tₘₐₓ is about 0.5 to about 1.5 hour, or about 1 hour; and/or
   T_{½} is less than 7 hours, about 2 to about 7 hours, about 3 to about 5 hours, or about 4 hours.
88. The composition for use of clause 87, wherein the corticosteroid is aclometasone, amcinomide, beclometasone, betamethasone, budesonide, ciclesonide, clobetasol, clobetasone, clocortolone, cloprednol, cortivazol, deflazacort, deoxycorticosterone, desonide desoximetasone, dexamethasone, diflorasone, diflucortolone, difluprednate, fluclorolone, fludrocortisone, fludroxycortide, flumetasone, flunisolide, fluocinolone acetonide, fluocinonide, fluocortin, fluocortolone, fluorometholone, fluperolone, fluticasone, fuprednidene, formocortal, halcinonide, halometasone, hydrocortisone aceponate, hydrocortisone buteprate, hydrocortisone butyrate, loteprednol, medrysone, meprednisone, methylprednisolone, methylprednisolone aceponate, mometasone, mometasone, paramethasone, prednicarbate, prednisone, prednisolone, prednylidene, remexolone, tixocortol, triamcinolone, ulobetasol, or a pharmaceutically acceptable salt or ester thereof.
89. The composition for use of clause 88, wherein the budesonide is administered to the individual in an amount of about 0.035 mg to about 4 mg per day, about 1 mg to about 4 mg per day, about 2 mg to about 4 mg per day, about 1.4 mg per day, or about 2.8 mg per day.
90. The composition for use of clause 87, wherein the corticosteroid is administered in combination with a preservative, a buffer, a surface active agent or a surfactant, an optional preservative, an optional flavoring agent, an optional sweetener, at least one additional excipient, and a liquid vehicle.
91. The composition for use of clause 87, wherein the corticosteroid is administered in combination with a edetate, citrate, polysorbate 80, an optional preservative, an optional flavoring agent, an optional sweetener, at least one additional excipient, and a liquid vehicle.
92. The composition for use of clause 87, wherein the composition is a solution, suspension, emulsion, syrup, slurry, dispersion, elixir, tonic, orally dissolving tablet, lozenge, powder, granules, micropellets, nanopellets, microparticles, nanoparticles, tablet, effervescent tablet, melting tablet, disintegrating tablet, orally disintegrating tablet, foam, gel, solid solution, solid formulation, solid disintegrating formulation, emulsion, liquid or semi-liquid solution, gum, wafer (e.g., dissolving or disintegrating), or a combination thereof, or a film or patch or the like.
93. An orally administered composition comprising an effective amount of a corticosteroid for use in providing one or more of, two or more of, three or more of, or all of the following:
   a. reducing eosinophilic infiltration of the esophagus;
   b. improving the visual appearance of the esophagus;
   c. epithelial improvement of the esophagus;
   d. reduction of fibrosis of the lamina propia;
   e. improvement in symptoms associated with esophageal inflammation.

## Claims

1. An orally administered composition comprising an effective amount of a corticosteroid for use in reducing eosinophilic infiltration of the esophagus in an individual in need thereof, or for inducing remission in an individual in need thereof.

2. The composition for use of claim 1, wherein, when eosinophilic infiltration of the esophagus is reduced, the individual has 1 or fewer eosinophils per high power field in the esophageal mucosa, or
wherein, when eosinophilic infiltration of the esophagus is reduced, the individual has 1 or fewer eosinophils per high power field in the distal esophageal mucosa, the mid esophageal mucosa, the proximal esophageal mucosa, or a combination thereof, optionally wherein when eosinophilic infiltration of the esophagus is reduced, the individual has 1 or fewer eosinophils per high power field in the distal esophageal mucosa, the mid esophageal mucosa, and the proximal esophageal mucosa, or
wherein, when eosinophilic infiltration of the esophagus is reduced, an endoscopy score is determined by:
(a) visually inspecting one or more sites of the esophagus for the following conditions 1) pallor and diminished vascular markings; 2) furrowing with thickened mucosa; 3) presence of white mucosal plaques; and 4) concentric rings or strictures;
(b) scoring each condition, and has an endoscopy score reduced by at least 50% compared to pretreatment score, or
wherein, when eosinophilic infiltration of the esophagus is reduced, a symptom score is determined by:
(a) determining if the individual exhibits a symptom in any one or more of the following conditions category or categories: 1) heartburn; 2) abdominal pain; 3) nocturnal awakening with symptoms; 4) nausea, regurgitation or vomiting; 5) anorexia or early satiety; or 6) dysphagia, odynophagia, or food impaction;
(b) scoring each condition category, and has a symptom score reduced by at least 50% compared to pretreatment score, or
wherein the individual is suffering from esophageal inflammation or symptoms thereof, or
wherein the individual is suffering from eosinophilic esophagitis, or
wherein the corticosteroid is a topically active corticosteroid, or
wherein the corticosteroid is aclometasone, amcinomide, beclometasone, betamethasone, budesonide, ciclesonide, clobetasol, clobetasone, clocortolone, cloprednol, cortivazol, deflazacort, deoxycorticosterone, desonide desoximetasone, dexamethasone, diflorasone, diflucortolone, difluprednate, fluclorolone, fludrocortisone, fludroxycortide, flumetasone, flunisolide, fluocinolone acetonide, fluocinonide, fluocortin, fluocortolone, fluorometholone, fluperolone, fluticasone, fuprednidene, formocortal, halcinonide, halometasone, hydrocortisone aceponate, hydrocortisone buteprate, hydrocortisone butyrate, loteprednol, medrysone, meprednisone, methylprednisolone, methylprednisolone aceponate, mometasone, paramethasone, prednicarbate, prednisone, prednisolone, prednylidene, remexolone, tixocortol, triamcinolone, ulobetasol, or a pharmaceutically acceptable salt or ester thereof, or a combination thereof, or
wherein the corticosteroid is budesonide, fluticasone, mometasone, desonide, ciclesonide, triamcinolone, beclomethasone, or a pharmaceutically acceptable ester thereof, or a combination thereof, optionally wherein the individual is less than 10 years old and the corticosteroid is administered in a composition having a total volume of about 5 to about 10 mL, or about 7 mL, or
wherein the corticosteroid is budesonide, or
wherein the individual is 12-17, 12-50, or 10 or more years old and the corticosteroid is administered in a composition having a total volume of about 8 to about 12 mL, or about 10 mL, or
wherein the corticosteroid is administered to the individual in an amount of about 0.035 mg or more per day, or
wherein the corticosteroid is administered to the individual in an amount of about 0.035 mg to about 10 mg per day, or
wherein the corticosteroid is administered to the individual in an amount of about 2 mg to about 4 mg per day, or
wherein the corticosteroid is administered to the individual in an amount of about 0.35 mg, 0.5 mg, 1.4 mg, 2 mg, or 4 mg per day, or
wherein the corticosteroid is administered to the individual at least once a day, or
wherein the corticosteroid is administered to the individual once a day, or
wherein the corticosteroid is administered to the individual twice a day.

3. The composition for use of claim 1, wherein the corticosteroid is administered to the individual in the form of a liquid composition, optionally wherein the composition comprises corticosteroid in an amount greater than 0.035 mg per mL, greater than 0.05 mg per mL or greater than 0.07 mg per mL of total volume of the composition, further optionally wherein: (i) the composition comprises about 0.1 mg to about 0.2 mg corticosteroid per milliliter of total volume of the composition, (ii) the composition comprises about 0.2 mg corticosteroid per milliliter of total volume of the composition, (iii) about 5 to about 15 milliliters of the composition is administered to the individual per dose, or (iv) about 7 to about 10 milliliters of the composition is administered to the individual per dose.

4. The composition for use of claim 1, wherein:
(i) the individual's morning cortisol levels during administration of the corticosteroid are in the range of 5-20 µg/dL or 8-14 µg/dL or does not fall below 5 µg/dL,
(ii) the reduction of eosinophilic infiltration of the esophagus is proportional to the amount of corticosteroid administered to the individual, or where greater reduction of eosinophilic infiltration of the esophagus occurs when a greater amount of corticosteroid is administered to the individual, or
(iii) the corticosteroid is administered to the individual in the form of a pharmaceutical composition comprising an effective amount of the corticosteroid and at least one pharmaceutically acceptable excipient, optionally wherein: (a) the pharmaceutical composition is in the form of a solution, suspension, emulsion, syrup, slurry, dispersion, elixir, tonic, orally dissolving tablet, lozenge, powder, granules, micropellets, nanopellets, microparticles, nanoparticles, tablet, effervescent tablet, melting tablet, disintegrating tablet, orally disintegrating tablet, foam, gel, solid solution, solid formulation, solid disintegrating formulation, emulsion, liquid or semi-liquid solution, gum, wafer (*e*.*g*., dissolving or disintegrating), or a combination thereof, or a film or patch or the like, or (b) the pharmaceutical composition comprises: a therapeutically effective amount of corticosteroid, a preservative, a buffer, a surface active agent or a surfactant, an optional preservative, an optional flavoring agent, an optional sweetener, at least one additional excipient, and a liquid vehicle, or a therapeutically effective amount of the corticosteroid, edetate, citrate, polysorbate 80, an optional preservative, an optional flavoring agent, an optional sweetener, at least one additional excipient, and a liquid vehicle.

5. A composition comprising a corticosteroid for use in treating esophageal inflammation in an individual in need thereof, comprising:
(a) orally administering to the individual according to a first daily dosing regimen comprising an amount of corticosteroid in an amount effective to reduce eosinophilic infiltration of the esophagus; and subsequently
(b) orally administering to the individual according to a second daily dosing regimen comprising an amount of corticosteroid in an amount effective to maintain a reduced level of eosinophilic infiltration of the esophagus or symptoms associated therewith.

6. The composition for use of claim 5, wherein:
(i) in step (a), the eosinophilic infiltration of the esophagus is reduced when the individual has 15 or fewer eosinophils per high power field in the esophageal mucosa,
(ii) in step (a), the eosinophilic infiltration of the esophagus is reduced when the individual has 15 or fewer, or 6 or fewer eosinophils per high power field in the esophageal mucosa,
(iii) in step (a), the eosinophilic infiltration of the esophagus is reduced when the individual has 6 or fewer, or 1 or fewer eosinophils per high power field in the esophageal mucosa,
(iv) when eosinophilic infiltration of the esophagus is reduced, an endoscopy score is determined by: (a) visually inspecting one or more sites of the esophagus for one or more of the following conditions 1) pallor and diminished vascular markings; 2) furrowing with thickened mucosa; 3) presence of white mucosal plaques; and 4) concentric rings or strictures; (b) scoring each condition and the individual has an endoscopy reduced by at least 50% compared to pretreatment score,
(v) the individual is suffering from eosinophilic esophagitis, or
(vi) the corticosteroid is aclometasone, amcinomide, beclometasone, betamethasone, budesonide, ciclesonide, clobetasol, clobetasone, clocortolone, cloprednol, cortivazol, deflazacort, deoxycorticosterone, desonide desoximetasone, dexamethasone, diflorasone, diflucortolone, difluprednate, fluclorolone, fludrocortisone, fludroxycortide, flumetasone, flunisolide, fluocinolone acetonide, fluocinonide, fluocortin, fluocortolone, fluorometholone, fluperolone, fluticasone, fuprednidene, formocortal, halcinonide, halometasone, hydrocortisone aceponate, hydrocortisone buteprate, hydrocortisone butyrate, loteprednol, medrysone, meprednisone, methylprednisolone, methylprednisolone aceponate, mometasone, paramethasone, prednicarbate, prednisone, prednisolone, prednylidene, remexolone, tixocortol, triamcinolone, ulobetasol, or a pharmaceutically acceptable salt or ester thereof, or a combination thereof, optionally wherein the corticosteroid is budesonide, fluticasone, mometasone, desonide, ciclesonide, triamcinolone, beclomethasone, or a pharmaceutically acceptable ester thereof, or a combination thereof, further optionally wherein the corticosteroid is budesonide, further optionally wherein: (1) the first daily amount is 2-6 mg per day, and (2) the second daily amount is 1-2 mg per day or the daily maintenance dose is 0.25-2 mg per day, or the first daily dosing regimen comprises an amount administered in a 1-2 mg dose twice per day and the second daily dosing regimen comprises an amount administered in a 1-2 mg dose once per day.

7. The composition for use of claim 5, wherein step (a) or (b) is repeated for at least six months or 12 months, or
wherein step (a) or (b) is repeated for at least one month, or
wherein the individual is 10 or more years old and the corticosteroid is administered in a composition having a total volume of about 8 to about 12 mL, or about 10 mL, or wherein the individual is less than 10 years old and the corticosteroid is administered in a composition having a total volume of about 5 to about 12 mL, or about 10 mL, or
wherein the corticosteroid in step (a) is administered to the individual twice a day, or
wherein the corticosteroid in step (b) is administered to the individual once a day.

8. The composition for use of claim 5, wherein:
(i) the corticosteroid is administered to the individual in the form of a liquid composition, optionally wherein (a) the composition comprises 0.035 mg or more, 0.5 mg or more, about 0.07 mg or more, or about 0.1 mg or more corticosteroid per milliliter, (b) the composition comprises about 0.1 mg to about 0.2 mg corticosteroid per milliliter, or about 0.2 mg corticosteroid per milliliter, or (c) about 5 to about 15 milliliters or about 7 to about 10 milliliters of the composition is administered to the individual per dose, or
(ii):
step (a) is repeated for about 3 months, and step (b) is repeated for about 3 months, or
step (a) is repeated for about 2 months, and step (b) is repeated for about 3 months, or
step (a) is repeated for about 3 months, and step (b) is repeated for about 2 months, or
step (a) is repeated for about 1 month, and step (b) is repeated for about 2 months, or
step (a) is repeated for about 1 month, and step (b) is repeated for about 1 month, or
step (a) is repeated for about 4 weeks, and step (b) is repeated for about 4 weeks, or
step (a) is repeated for about 2 weeks, and step (b) is repeated for about 2 weeks, or
step (a) is repeated for about 3 months, and step (b) is repeated indefinitely, or
step (a) is repeated for about 2 months, and step (b) is repeated indefinitely, or
step (a) is repeated for about 1 month, and step (b) is repeated indefinitely, or
step (a) is repeated for about 4 weeks, and step (b) is repeated indefinitely, or
step (a) is repeated for about 2 weeks, and step (b) is repeated indefinitely.

9. The composition for use of claim 5, wherein the corticosteroid is administered to the individual in the form of a pharmaceutical composition comprising an effective amount of the corticosteroid and at least one pharmaceutically acceptable excipient, optionally wherein: (i) the pharmaceutical composition is in the form of a solution, suspension, emulsion, syrup, slurry, dispersion, elixir, tonic, orally dissolving tablet, lozenge, powder, granules, micropellets, nanopellets, microparticles, nanoparticles, tablet, effervescent tablet, melting tablet, disintegrating tablet, orally disintegrating tablet, foam, gel, solid solution, solid formulation, solid disintegrating formulation, emulsion, liquid or semi-liquid solution, gum, wafer (e.g., dissolving or disintegrating), or a combination thereof, or a film or patch or the like, or (ii) the pharmaceutical composition comprises: a therapeutically effective amount of corticosteroid, a preservative, a buffer, a surface active agent or a surfactant, an optional preservative, an optional flavoring agent, an optional sweetener, at least one additional excipient, and a liquid vehicle, or a therapeutically effective amount of the corticosteroid, edetate, citrate, polysorbate 80, an optional preservative, an optional flavoring agent, an optional sweetener, at least one additional excipient, and a liquid vehicle.

10. A composition comprising a corticosteroid for use in treating esophageal inflammation in an individual in need thereof, comprising:
(a) orally administering to the individual according to a first daily dosing regimen comprising an amount of corticosteroid in an amount effective to reduce the individual's eosinophil count to 6 or fewer or 1 or fewer eosinophils per high power field in the esophageal mucosa; and
(b) orally administering to the individual according to a second daily dosing regimen comprising an amount of corticosteroid in an amount effective to maintain the individual's eosinophil count at 6 or fewer or 1 or fewer eosinophils per high power field in the esophageal mucosa.

11. A composition comprising a corticosteroid for use in treating esophageal inflammation in an individual in need thereof, comprising:
(a) orally administering to the individual according to a first daily dosing regimen comprising an amount of corticosteroid in an amount effective to reduce eosinophilic infiltration of the esophagus;
(b) orally administering to the individual according to a second daily dosing regimen comprising an amount of corticosteroid in an amount effective to further reduce eosinophilic infiltration of the esophagus; and subsequently
(c) orally administering to the individual according to a third daily dosing regimen comprising an amount of corticosteroid in an amount effective to maintain a reduced level of eosinophilic infiltration of the esophagus.

12. The composition for use of claim 11, wherein in step (a), the eosinophilic infiltration of the esophagus is reduced when the individual has 23 or fewer, or 15 or fewer eosinophils per high power field in the esophageal mucosa, or
wherein in step (b), the eosinophilic infiltration of the esophagus is further reduced when the individual has 15 or fewer, or 6 or fewer, or 1 or fewer eosinophils per high power field in the esophageal mucosa, or
wherein, when eosinophilic infiltration of the esophagus is reduced, wherein an endoscopy score is determined by: (a) visually inspecting one or more sites of the esophagus for one or more of the following conditions 1) pallor and iminished vascular markings; 2) furrowing with thickened mucosa; 3) presence of white mucosal plaques; and 4) concentric rings or strictures; (b) scoring each condition, and the individual has an endoscopy score reduced by at least 50% compared to pretreatment score, or
wherein, when eosinophilic infiltration of the esophagus is reduced, a symptom score is determined by: (a) determining if the individual exhibits a symptom in any one or more of the following conditions category or categories: 1) heartburn; 2) abdominal pain; 3) nocturnal awakening with symptoms; 4) nausea, regurgitation or vomiting; 5) anorexia or early satiety; or 6) dysphagia, odynophagia, or food impaction; (b) scoring each condition category, and has a symptom score reduced by at least 50% compared to pretreatment score, or wherein, when eosinophilic infiltration of the esophagus is reduced, a LP score is determined by:
(a) determining whether the individual's esophagus exhibits lamina propria (LP) fibrosis; and (b) scoring the condition; and has a LP score reduced by at least 50% compared to pretreatment score, or
wherein the individual is suffering from eosinophilic esophagitis, or
wherein the corticosteroid is aclometasone, amcinomide, beclometasone, betamethasone, budesonide, ciclesonide, clobetasol, clobetasone, clocortolone, cloprednol, cortivazol, deflazacort, deoxycorticosterone, desonide desoximetasone, dexamethasone, diflorasone, diflucortolone, difluprednate, fluclorolone, fludrocortisone, fludroxycortide, flumetasone, flunisolide, fluocinolone acetonide, fluocinonide, fluocortin, fluocortolone, fluorometholone, fluperolone, fluticasone, fuprednidene, formocortal, halcinonide, halometasone, hydrocortisone aceponate, hydrocortisone buteprate, hydrocortisone butyrate, loteprednol, medrysone, meprednisone, methylprednisolone, methylprednisolone aceponate, mometasone, paramethasone, prednicarbate, prednisone, prednisolone, prednylidene, remexolone, tixocortol, triamcinolone, ulobetasol, or a pharmaceutically acceptable salt or ester thereof, or a combination thereof, optionally wherein the corticosteroid is budesonide, fluticasone, mometasone, desonide, ciclesonide, triamcinolone, beclomethasone, or a pharmaceutically acceptable ester thereof, or a combination thereof, further optionally wherein the corticosteroid is budesonide, further optionally wherein: the first daily dosing regimen comprises an amount of 1-2 mg per day, the second daily dosing regimen comprises an amount of 2-6 mg per day, and the third daily dosing regimen comprises an amount of 0.035-2 mg per day, or the first daily dosing regimen comprises an amount administered in a 1-2 mg dose once per day, the second daily dosing regimen comprises an amount administered in a 2-3 mg dose twice per day, and the third daily dosing regimen comprises an amount administered in a 1-2 mg dose once per day.

13. The composition for use of claim 11, wherein:
step (a) is repeated for about 3 months, and step (b) is repeated for about 3 months, or
step (a) is repeated for about 2 months, and step (b) is repeated for about 3 months, or
step (a) is repeated for about 3 months, and step (b) is repeated for about 2 months, or
step (a) is repeated for about 1 month, and step (b) is repeated for about 2 months, or
step (a) is repeated for about 1 month, and step (b) is repeated for about 1 month, or
step (a) is repeated for about 4 weeks, and step (b) is repeated for about 4 weeks, or
step (a) is repeated for about 2 weeks, and step (b) is repeated for about 2 weeks, or
wherein step (a), (b), or (c) is repeated for at least one month, or at least six months, or
wherein the individual is 10 or more years old and the corticosteroid is administered in a composition having a total volume of about 8 to about 12 mL, or about 10 mL, or
wherein the individual is less than 10 years old and the corticosteroid is administered in a composition having a total volume of about 5 to about 12 mL, or about 7 mL, or
wherein the corticosteroid in step (b) is administered to the individual twice a day, or
wherein the corticosteroid in step (a) or (c) is administered to the individual once a day, or
wherein the corticosteroid is administered to the individual in the form of a liquid composition, optionally wherein: (i) the composition comprises 0.035 mg or more, 0.5 mg or more, about 0.07 mg or more, or about 0.1 mg or more corticosteroid per milliliter, (ii) the composition comprises about 0.1 mg to about 0.2 mg corticosteroid per milliliter, (iii) the composition comprises about 0.2 mg corticosteroid per milliliter, or (iv) about 5 to about 15 milliliters or about 7 to about 10 milliliters of the composition is administered to the individual per dose.

14. The composition for use of claim 11, wherein the corticosteroid is administered to the individual in the form of a pharmaceutical composition comprising an effective amount of the corticosteroid and at least one pharmaceutically acceptable excipient, optionally wherein: (i) the pharmaceutical composition is in the form of a solution, suspension, emulsion, syrup, slurry, dispersion, elixir, tonic, orally dissolving tablet, lozenge, powder, granules, micropellets, nanopellets, microparticles, nanoparticles, tablet, effervescent tablet, melting tablet, disintegrating tablet, orally disintegrating tablet, foam, gel, solid solution, solid formulation, solid disintegrating formulation, emulsion, liquid or semi-liquid solution, gum, wafer (*e*.*g*., dissolving or disintegrating), or a combination thereof, or a film or patch or the like, or (ii) the pharmaceutical composition comprises: a therapeutically effective amount of corticosteroid, a preservative, a buffer, a surface active agent or a surfactant, an optional preservative, an optional flavoring agent, an optional sweetener, at least one additional excipient, and a liquid vehicle, or a therapeutically effective amount of the corticosteroid, edetate, citrate, polysorbate 80, an optional preservative, an optional flavoring agent, an optional sweetener, at least one additional excipient, and a liquid vehicle.

15. A composition comprising a corticosteroid for use in treating esophageal inflammation in an individual in need thereof, comprising:
(a) orally administering to the individual according to a first daily dosing regimen comprising an amount of corticosteroid in an amount effective to reduce the individual's eosinophil count to 23 or fewer or 15 or fewer eosinophils per high power field in the esophageal mucosa; and
(b) orally administering to the individual according to a second daily dosing regimen comprising an amount of corticosteroid in an amount effective to reduce the individual's eosinophil count to 15 or fewer, 6 or fewer or 1 or fewer eosinophils per high power field in the esophageal mucosa; and subsequently
(c) orally administering to the individual according to a third daily dosing regimen comprising an amount of corticosteroid in an amount effective to maintain the individual's eosinophil count at 15 or fewer, 6 or fewer or 1 or fewer eosinophils per high power field in the esophageal mucosa.

16. An orally administered composition comprising an effective amount of a corticosteroid for use in reducing eosinophilic infiltration of the esophagus in an individual in need thereof, or for inducing remission in an individual in need thereof, wherein plasma:
AUC₀₋₈ₕ is less than 4000 h^{∗}pg/mL, about 2000 to about 4000 h^{∗}pg/mL, about 2500 to about 3000 h^{∗}pg/mL, or about 2700 h^{∗}pg/mL; and/or Cₘₐₓ is less than 1500 pg/mL, about 500 to about 1500 pg/mL, about 600 to about 900 pg/mL, or about 700 pg/mL; and/or Tₘₐₓ is about 0.5 to about 1.5 hour, or about 1 hour; and/or T_{½} is less than 7 hours, about 2 to about 7 hours, about 3 to about 5 hours, or about 4 hours,
optionally wherein: (i) the corticosteroid is aclometasone, amcinomide, beclometasone, betamethasone, budesonide, ciclesonide, clobetasol, clobetasone, clocortolone, cloprednol, cortivazol, deflazacort, deoxycorticosterone, desonide desoximetasone, dexamethasone, diflorasone, diflucortolone, difluprednate, fluclorolone, fludrocortisone, fludroxycortide, flumetasone, flunisolide, fluocinolone acetonide, fluocinonide, fluocortin, fluocortolone, fluorometholone, fluperolone, fluticasone, fuprednidene, formocortal, halcinonide, halometasone, hydrocortisone aceponate, hydrocortisone buteprate, hydrocortisone butyrate, loteprednol, medrysone, meprednisone, methylprednisolone, methylprednisolone aceponate, mometasone, mometasone, paramethasone, prednicarbate, prednisone, prednisolone, prednylidene, remexolone, tixocortol, triamcinolone, ulobetasol, or a pharmaceutically acceptable salt or ester thereof, optionally wherein the budesonide is administered to the individual in an amount of about 0.035 mg to about 4 mg per day, about 1 mg to about 4 mg per day, about 2 mg to about 4 mg per day, about 1.4 mg per day, or about 2.8 mg per day, (ii) the corticosteroid is administered in combination with a preservative, a buffer, a surface active agent or a surfactant, an optional preservative, an optional flavoring agent, an optional sweetener, at least one additional excipient, and a liquid vehicle, (iii) the corticosteroid is administered in combination with a edetate, citrate, polysorbate 80, an optional preservative, an optional flavoring agent, an optional sweetener, at least one additional excipient, and a liquid vehicle, or (iv) the composition is a solution, suspension, emulsion, syrup, slurry, dispersion, elixir, tonic, orally dissolving tablet, lozenge, powder, granules, micropellets, nanopellets, microparticles, nanoparticles, tablet, effervescent tablet, melting tablet, disintegrating tablet, orally disintegrating tablet, foam, gel, solid solution, solid formulation, solid disintegrating formulation, emulsion, liquid or semi-liquid solution, gum, wafer (*e*.*g*., dissolving or disintegrating), or a combination thereof, or a film or patch or the like.

17. An orally administered composition comprising an effective amount of a corticosteroid for use in providing one or more of, two or more of, three or more of, or all of the following:
a. reducing eosinophilic infiltration of the esophagus;
b. improving the visual appearance of the esophagus;
c. epithelial improvement of the esophagus;
d. reduction of fibrosis of the lamina propia;
e. improvement in symptoms associated with esophageal inflammation.
